# EUROPEAN PATENT SPECIFICATION

(11) **EP 2 934 580 B1**
(45) Date of publication and mention of the grant of the patent: **18.09.2019**
(21) Application number: 13824205.2
(22) Date of filing: 17.12.2013
(51) Int. Cl.: A61K 39/112, A61K 39/395, A61K 39/00, A61P 31/04

(54) **A POLYVALENT COMBINED IMMUNISING AND/OR THERAPEUTIC PREPARATION FOR USE IN BACTERIAL INFECTIONS OR FOOD POISONING, PARTICULARLY SALMONELLOSIS, A METHOD FOR PRODUCTION OF THIS PREPARATION, ITS USE AND A VACCINE COMPRISING THIS PREPARATION**
POLYVALENTES KOMBINIERTES IMMUNISIERENDES UND/ODER THERAPEUTISCHES PRÄPARAT ZUR VERWENDUNG BEI BAKTERIELLEN INFEKTIONEN ODER LEBENSMITTELVERGIFTUNG, INSBESONDERE SALMONELLOSE, VERFAHREN ZUR HERSTELLUNG DIESES PRÄPARATS, DESSEN VERWENDUNG UND IMPFSTOFF MIT DIESEM PRÄPARAT
PRÉPARATION POLYVALENTE COMBINÉE D'IMMUNISATION ET/OU DE THÉRAPIE POUR UTILISATION LORS D'INFECTIONS BACTÉRIENNES OU D'INTOXICATIONS ALIMENTAIRES, PARTICULIÈREMENT ASSOCIÉES À LA SALMONELLOSE, PROCÉDÉ DE PRODUCTION DE CETTE PRÉPARATION, SON UTILISATION ET VACCIN COMPRENANT CETTE PRÉPARATION

(30) Priority: 20.12.2012 PL 40215212
(43) Date of publication of application: 28.10.2015
(73) Proprietor: Zaklad Badawczo-Wdrozeniowy Osrodka Salmonella "Immunolab" Sp. z o.o., 81-451 Gdynia (PL)
(72) Inventor: GLOSNICKA, Renata, 80-460 Gdansk (PL); GLOSNICKI, Krzysztof, 83-050 Babidól (PL); LIEDER, Dorota, 83-050 Babidól (PL)
(74) Representative: Dargiewicz, Joanna
(86) International application number: PCT/IB2013/061054
(87) International publication number: WO 2014/097154

(56) References cited:
- WO-A1-90/08184
- WO-A1-98/14212
- GB-A- 1 416 544
- PL-B1- 169 172
- GLOSNICKA R. ET AL.: "Immunovac - poliwalentna szczepionka przeciw salmonellozom kur", MEDYCYNA WET., vol. 61, no. 10, 2005, pages 1105-1109, XP9176901,
- Anonymous: "Prof. Glosnicka heads a succesful startup company in the growing biotech industry", , 19 October 2009 (2009-10-19), XP055107695, Retrieved from the Internet: URL:http://www.scanbalt.org/press/news+arc hive/view?id=2162 [retrieved on 2014-03-13]

## Description

An object of the invention is a safe and effective immunising and/or therapeutic preparation for use in bacterial infections and food poisoning, particularly salmonellosis, a method for production and use thereof. The invention relates also to a vaccine against bacterial infections, comprising above mentioned immunising and/or therapeutic preparation.

Salmonelloses are zoonoses, which may be transmitted directly or indirectly between animals and humans. *Salmonella* bacteria are quite common in intestines of healthy birds and mammals, in food, and most often there are found in eggs and raw pork, turkey and chicken meat. This disease may spread on humans via contaminated food.

The increasing demand for food, rapid development of large-scale poultry farms and industry scale food production in the last century were the direct causes of salmonellosis pandemics.

The fight against salmonelloses is extremely complicated. On one hand, we have the phenomenon of serological diversity of these bacteria, and on the other hand the great prevalence of *Salmonella* in nature. They are resistant to the most of the known antibiotics and the attempts of their elimination under farming conditions through immunisation are associated with the need to develop a vaccine against each of the serovars of "special importance for public health" (Barrow, P. A. (2007) "Salmonella infections: immune and non-immune protection with vaccines", Avian Pathology, 36: 1,1-13).

*Salmonella* bacteria belong to the *Enterobacteriaceae* family. Based on the genetic features and biochemical properties *Salmonella* was divided into two species: *Salmonella enterica* and *Salmonella bongori.* Within *Salmonella enterica* there are following subspecies: *enterica, salamae, arizonae, diarizonae, houtenae* and *indica.* (Grimont, P.A.D. & Weill F-X, Antigenic formulae of the Salmonella serovars, 2007, 9th edition, WHO Collaborating Centre for Reference and Research on Salmonella).

Until now, there have been 2579 serotypes of *Salmonella* described within the *Salmonella* genus. These serotypes have been classified based on the presence of somatic (O), capsular (Vi) and flagellar (H) antigens on their surface. This scheme, called Kauffmann-White scheme shows the classification of *Salmonella* serovar antigenic structure, and consists of 63 serogroups. Only a rather limited number of *Salmonella* serovars are common.

Lipopolysaccharide (antigen O, endotoxin) plays an important role in the bacterial cell. It is one of the pathogenicity determinants for humans and animals. Lipopolysaccharide is a heteropolymer consisting of three structurally diverse regions: lipid A, core oligosaccharide and O-specific polysaccharide chain. O-specific polysaccharide chain (OPS) forms the distal part of the lipopolysaccharide. It is the most variable fragment and it is this part of the endotoxin, which stimulates the antibody production. This lipopolysaccharide is called an endotoxin, because it causes severe complications in case of bacterial infections, with such symptoms as sepsis and septic shock. Research carried out for many years allowed the inventors to obtain some very important information on the basic immunogenic properties of the selected structures of these antigens. This research was the basis for the composition of a polyvalent, multicomponent vaccine to control salmonelloses. (Publications: Kumirska J., Szafranek J., Czerwicka M., Go 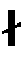 ebiowski M., Paszkiewicz M., Dziadziuszko H., Kunikowska D., Stepnowski P., Smith degradation of the O-antigenic polysaccharide of *Salmonella Dakar.* structural studies of the products, *Carbohydr Res.* 2008, 343(6): 1120-1125; J. Gajdus, R. G 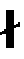 ośnicka, J. Szafranek. I-Rz 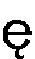 dowa struktura antygenów O bakterii *Salmonella* (Primary structure of *Salmonella* spp. O-antigens), *Wiadomości Chemiczne,* 2006, 60:9-10); Szafranek J., Kumirska J., Czerwicka M., Kunikowska D., Dziadziuszko H., G 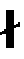 ośnicka R., Structure and heterogeneity of the O-antigen chain of *Salmonella Agona* lipopolysaccharide, *FEMS Immunol. Med. Microbiol.* 2006, 48: 223-236; Szafranek J., Kaczyńska M., Kaczyński J., Gajdus J., Czerwicka M., Dziadziuszko H., G 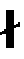 ośnicka R., Structure of the polysaccharide O-Antigen of *Salmonella* Aberdeen (O:11), *Polish J. Chem.* 2003, 77:1135-1140; H. Dziadziuszko, D. Kunikowska, R. G 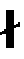 ośnicka, J. Gajdus, Z. Kaczyński and J. Szafranek, Immunological and chemical studies of *Salmonella haarlem* somatic antigen epitopes. II. Serological investigation, *FEMS Immunology and Medical Microbiology* 1998, 21(4): 253-259; J. Szafranek, J. Gajdus, Z. Kaczyński, H. Dziadziuszko, D. Kunikowska, R Gtośnicka, T. Yoshida, J. Vihanto and K. Pihlaja, Immunological and chemical studies of Salmonella haarlem somatic antigen epitopes. I. Structural studies of O-antigen., FEMS Immunology and Medical Microbiology 1998, 21(4): 243-252).

Flagellar antigen is a surface antigen that is related with the presence of flagella on the surface of *Salmonella.* There are particularly abundant in broth culture and in Sven Gard Agar. Flagellar antigens, H, of *Salmonella* are usually biphasic. Most of bacteria of the *Salmonella* genus move with flagelli.

On the other hand, the Vi antigen is localised in the bacterial cell envelope. It is composed of a polysaccharide, poly-N-acetylamino galacturonic acid. Vi antigen is produced by only some of *Salmonella* strains.

*Salmonella* serovars belonging to the *enterica* subspecies is a group of pathogens varying in their preferences of host, in which they can cause a disease. These host preferences may be very narrow, as in the case of *S.* Typhi infecting only humans and primates, or may be of wider range (including humans and many animal species), as in case of infections caused by *Salmonella* Typhimurium and *Salmonella* Enteritidis.

The vast majority of pathogenic serovars associated with human infections belong to *Salmonella* sp. *enteric* ssp. *enterica.* In 2006 - 2009, among the most prevalent serovars in humans in Europe were: S. Enteritidis, S. Typhimurium, S. Infantis, S. Bovismorbificans, S. Hadar, S. Virchow, S. Derby, S. Newport, S. Stanley, S. Agona, S. Kentucky and S. Saintpaul. In recent years, a monophasic variant of S. Typhimurium strain appeared, such as (e.g., 1,4, [5], 12: i: -) in pigs, cattle, pets and humans, what is a new threat to the environment and thus a risk of contamination of feed ingredients, what increases the possibility of biosecurity breach. (EFSA Journal 2011;9(7):2106).

The European Surveillance System (EFSA and ECDC, 2011) in 2009 reported 108,614 confirmed cases of human salmonellosis in the 27 EU Member States. Incidence rate was 23.7 cases per 100,000 individuals, (from 2.1 in Portugal, 22.3 in Poland to 100.1 in the Czech Republic), European Centre for Disease Prevention and Control. Annual Epidemiological Report 2011.

As in previous years, *S.* Enteritidis and *S.* Typhimurium were the most frequently reported serovars (75.6% of all reported serovars). EFSA estimates that the total economic burden associated with salmonelloses in humans can be up to 3 milliard EUR per year.

Chicken flock infection prevalence in 26 countries, on average was 26%. Most of the infected flocks were in Portugal - 79,5%. Poland was on the second place with 76.2% of infected chicken flocks. Next countries on the list were Spain, Czech Republic and Greece; in these countries chicken infection prevalence was more than 50% (EFSA Journal (2007) 98, 1-85).

Protective vaccinations are the only acceptable way to reduce and/or eradicate *Salmonella* infections in farm birds. In accordance with the directives of the European Union the obligation to control diseases applies to five serovars (*Salmonella* Enteritidis, *Salmonella* Typhimurium, *Salmonella* Hadar, *Salmonella* Virchow and *Salmonella* Infantis).

Protective vaccinations against *Salmonella* are extremely popular nowadays, and thus one uses:

### 1. Vaccines containing inactivated (killed) bacteria

In experimental and field research various types of inactivated vaccines have been used. Such vaccines may contain bacteria inactivated with formaldehyde, acetone, glutaraldehyde or heat. In some vaccines, oil adjuvants, aluminium hydroxide and other compounds were used to stimulate the immune system. The vaccines containing killed bacteria, used for subcutaneous or intramuscular injections, have the ability to elicit a strong immune response, but produce little or unequivocal protection (Barrow, P.A. 2007). These vaccines typically contain one or two serovars e.g., *S.* Enteritidis and *S.* Typhimurium, and are given between the 4th and 6th week of life. Also recommended is to administer these vaccines two times.

### 2. Live vaccines

The current vaccines may contain live attenuated, genetically modified bacteria. The use of oral vaccines containing live genetically modified bacteria against salmonelloses stimulates the local intestinal immunity and also helps to reduce excretion of bacteria in the treated animals. The use of these vaccines is subject to certain limitations (COMMISSION REGULATION (EC) No. 1177/2006, below). Administering live bacteria in the vaccine to the animals is associated with a strong response to endotoxin; there were cases of illness and death of animals. Such vaccines may not be used in case of an acute infection in the flock, and they do not produce immunity in very young animals. Application of genetically modified vaccines in the first day of life did not protect the chickens form liver and spleen colonization by the infectious strain (Van Immerseel 2002. Early protection against *Salmonella* infection in chickens by modification of the initial interactions host-pathogen - doctoral dissertation). This phenomenon is explained by the immaturity of the immune system in birds. Maturation of the immune system in birds after hatching takes 3 weeks. All known vaccines are used in animals from age of 3-4 weeks. Manufacturers recommend also administering these vaccines two or three times.

For many years there are known bactericidal and protective properties of antisera and antibodies obtained from the egg yolk of birds (chickens or ducks) immunised with bacteria or viruses. (Peltra, C. et al. 1994, Taylor P.W., 1998, Yokoyama, H. et al., 1998, Raja Chalghoumi, R., Beckers, Y., Portetelle, D., Théwis, A. Biotechnol. Agron. Soc. Environ. 2009 13(2), 295-308, Meenatchisundaram. S, Michael, A., Subbraj, T., Diraviam, T., Shanmugam, V. International Journal of Drug Development & Research 2011 (vol. 3), Jann Hau and Coenraad F. M. Hendriksen, Refinement of Polyclonal Antibody Production by Combining Oral Immunization of Chickens with Harvest of Antibodies from the Egg Yolk, ILAR Journal, vol. 46, no. 3, 2005).

Unfortunately, according to epizoosociological and epidemiological studies conducted between 2010 and 2011, the intended results to reduce the number of infections on poultry farms with serovars of *Salmonella* Enteritidis and *Salmonella* Typhimurium were not obtained.

Accordingly, there are several studies conducted to develop a vaccine against salmonelloses. Thus, international patent application WO 2011092185 A1 describes new mutants of *Salmonella,* a method for their production and vaccines containing these mutants, wherein said *Salmonella* mutants are characterized by the fact that they elicit an antibody response that can be distinguished from the antibody response elicited by the wild type strains. The invention relates to the use of these mutant strains, as a serological marker for the vaccination of animals, especially mammals and birds. A vaccine, which is a serological marker, induces long-lasting immunisation of animals to a wide range of *Salmonella* strains.

The international patent application WO 2007112518 A1 discloses a double or triple attenuated mutant strains of bacteria causing infections in animals, such as *Salmonella* enterica and/or *Escherichia coli.* The mutants of the invention contain at least one genetic modification of the first type and at least one genetic modification of the second type. The invention relates also to the live attenuated vaccines based on these mutants for use in the prevention of salmonelloses and infections caused by pathogenic *E*. *coli* in animals. Another vaccine, comprising at least one attenuated *Salmonella* strain, is disclosed in international patent application WO 2008073891 A2.

The Russian patent RU 2377015 C1 discloses an inactivated vaccine against salmonellosis in pigeons, which includes strains of *Salmonella* Typhimurium M-5v t-DEP, *Salmonella* Typhimurium D-1v t-DEP and *Salmonella* Enteritidis 25 Yav e-DEP inactivated with formalin and embedded in polyethylene glycol for intramuscular application.

The Ukrainian Patent No. AU 12945 U discloses a combined, inactivated, concentrated Polyaviasan type vaccine for poultry, among other things against salmonellosis, which contains soluble cell wall antigens of *Escherichia coli, Salmonella* Enteritidis, *Salmonella* typhimurium, *Salmonella* Gallinarum, *Clostridium perfringens* A and C type in an optimal ratio with an immunostimulatory component being a medicinal herb extract, which stimulates the synthesis of antibodies, and other conventional ingredients. The vaccine can be administered to young poultry, even a one-day old.

Whereas, an Ukrainian Patent No. AU 12941 U discloses a combined, inactivated, concentrated Velshicolisalm type vaccine for animals, among other things against salmonellosis, which contains soluble cell wall antigens of *Escherichia coli* (6 strains), *Salmonella enteritidis, Salmonella dublin, Salmonella typhi suis, Salmonella typhi murium, Salmonella cholerae suis, Clostridium perfringens* A and C type, in an optimal ratio, with an immunostimulatory component being a medicinal herb extract, which stimulates the synthesis of antibodies, and other conventional ingredients.

The Polish patent PL 206377 B1 describes the use of bacteria of the *Salmonella* genus, which wild type produces flagella (these bacteria are not able to induce antibody production against at least one antigenic determinant of flagellin or flagellum) and an adjuvant for the preparation of an inactivated vaccine for the protection of humans and animals against *Salmonella* infection or pathological effects of such infection. The vaccine of the above mentioned invention can be administered to animals or humans, among other things, intranasally, by spraying, intradermally, subcutaneously, orally, in form of an aerosol or intramuscularly. In the case of poultry, the vaccine is administered to wings or as eye drops.

However, the Polish patent PL 186873 B1 discloses a method for production a trivalent vaccine for birds against salmonellosis, paramyxovirosis and mycoplasmosis, especially for chickens and pigeons, characterized in that the heat-inactivated antigens of *Salmonella* rods are mixed with formalin-inactivated antigens of La-Sota strain of PMV-1 virus and formalin-inactivated cells of *Mycoplasma gallisepticum,* then Tween 80 is added and emulsified with an oil adjuvant.

In: Medycyna Wet. 2005, 61 (10), 1105 - 1109, G 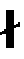 ośnicka R., Dziadziuszko H., Kunikowska D., Lis M., Wicha M. Heleski M., Tokarska-Pietrzak E., Strzatkowski L. Bugajak P. "Immunovac - polyvalent vaccine against salmonellosis in hens" disclosed a new combined, polyvalent vaccine for salmonellosis control in hens. Immunovac contained 8 *Salmonella* strains bound to rabbit antibodies. Immunovac vaccine was developed in 1989. This vaccine was a suspension of inactivated *Salmonella* strains and rabbit antibodies, intended for laying hens and breeding flocks for salmonellosis prevention. This preparation, in form of a suspension, consisted of a complex of 8 *Salmonella* strains bound to their specific antibodies. Bacteria were inactivated with formaldehyde and suspended in sodium chloride (8.5 g/cm³) containing 0.02% formaldehyde. One millilitre of the suspension contained approximately 3.5x10¹¹ of bacteria: S. Gallinarum - 0.4x10¹¹, S. Enteritidis - 1.5x10¹¹, S. Dublin - 0.4x10¹¹, S. Typhimurium - 0.8x10¹¹, S. Heidelberg - 0.1x10¹¹, S. Choleraesuis - 0.1x10¹¹, *S.* Kentucky - 0.1x10¹¹ and S. Senftenberg - 0.1x10¹¹. The bacteria were bound to polyclonal antibodies obtained from sera of rabbits immunised with somatic antigens of *Salmonella* from: B, C₁, C₂-₃ and D₁ groups. A detailed method of Immunovac vaccine preparation is a subject matter of a patent (PL 196172).

To study the antimicrobial activity, an ability to induce resistance to infection in mice, immunisation of rabbits and to stimulate cellular and humoral activity a vaccine produced on a laboratory scale was used.

### These studies included:

- *In vitro* evaluation of the antimicrobial activity of the vaccine;
- Evaluation of the ability to stimulate cell activity with splenocytes of mice vaccinated *per os.*

To determine the protective properties of the vaccine for hens, the Immunovac vaccine produced on a large scale (by Biowet- Pu 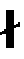 awy) was used. Hens come from flocks reared on farms and received the vaccine four times in drinking water.

Summing up the results, it was found that the Immunovac vaccine showed the ability to activate the immune system. It caused a humoral and cellular immunity stimulation. It provided 100% protection of mice against *per os* infection with *S.* Enteritidis strain and 80% protection of hens against *S.* Gallinarum bacteria given intramuscularly. It had a bacteriostatic effect on *S.* Enteritidis and *S.* Typhimurium in *in vitro assays.* It demonstrated its ability to stimulate lymphocyte proliferation, and also the high activity of inducing specific receptors on the lymphocytes of the vaccinated mice.

Studies have shown in rabbits the presence of antibodies to specific strains contained in the vaccine. The commercial Idexx assay (commercial assay for serological identification of *Salmonella* infections in birds, currently not used) did not showed any increased level of antibodies in vaccinated hens. A polyvalent combined Immunovac vaccine is a safe, easy-to-use and effective preparation for the control of salmonellosis in hens.

### The method of Immunovac vaccine preparation developed in 1989

**Bacteria** - each strain is collected separately, one copy each from described museum repository, and grown in the agar medium.

Rejuvenated starting basic mass is transferred to the broth and incubated for 20 hours at 37 C. Broth bacterial suspension is plated on glass plates containing enriched agar medium. The bacteria are cultured for 20 hours at 37 C, then washed off and transferred to a sterile flask. Before proceeding to the next steps, the bacterial suspension should be centrifuged and the number of bacteria determined by serial dilutions and colony counting. One gram of bacteria contains about 3.5x10¹³ colony forming units (cfu). To the bacterial suspension formaldehyde is added to concentration of 0.4% and it is left at 37 C for 24 hours in order to kill bacteria.

**Polyclonal antibodies obtained from sera of rabbits immunised with somatic antigens of *Salmonella* from B, C₁, C₂₋₃ and D₁ groups were bound to bacteria.** The binding procedure is carried out as follows: After verifying the sterility of inactivated bacteria, the rabbit serum is added to the centrifuged mass per each 1 gram of bacterial pellet (15 ml of the appropriate serum diluted with sodium chloride, 1:3). The mixture is incubated for 20 hours at 37 C, centrifuged and after resuspension of the pellet in sodium chloride, stored at 4C.

Components of the vaccine - inactivated bacteria mixed with antibodies, after conducting adequate controls, were mixed in the following proportions:
- *S.* Enteritidis - 15 g
- *S.* Gallinarum - 4 g;
- *S.* Dublin - 4 g;
- *S.* Typhimurium - 8 g;
- *S.* Heidelberg - 1 g;
- *S.* Choleraesuis - 1 g;
- *S.* Kentucky - 1 g;
- *S.* Senftenberg - 1 g

The prepared bacterial suspension was filled up with sodium chloride, added with formaldehyde to a concentration of 0.02%, poured into 100 ml bottles and stored at 2 to 8 C. The final product, in addition to other prescribed characteristics, must be sterile and contain a specified amount of antibodies (measured by ELISA).

Except the evaluations described in that publication, the inventors performed:

### Evaluation of the immunogenic activity of Immunovac vaccine in hens

Experiments were performed to determine the specific immune response after Immunovac vaccine administration. The level of IgG anti*-Salmonella* antibodies in chicken blood before and after full 4-time vaccination cycle was determined. The chickens were immunised orally with the vaccine for the first time in the second week of their life. Subsequent doses were given to birds *per os* at weekly intervals. The breeding flock population was 14.000, and the birds for the blood test were selected randomly. Group 1 consisted of 15 animals, group 2 - of 8 animals, a the control group of non-vaccinated chickens consisted of 10 animals.

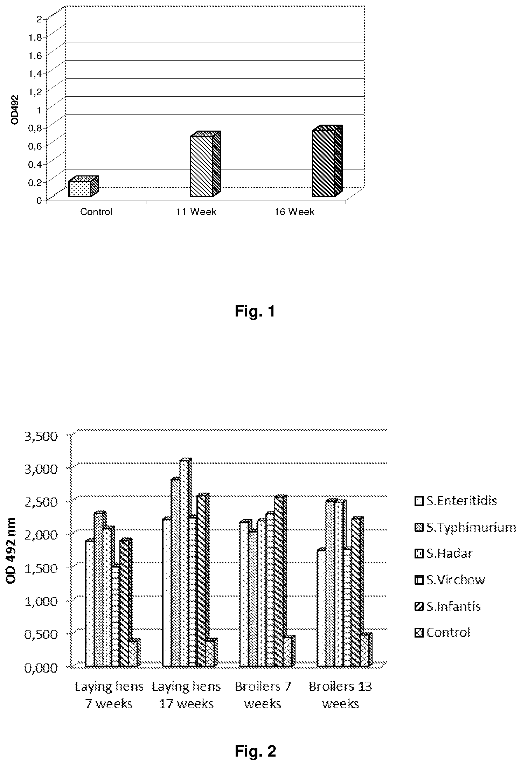

The level of IgG anti*-Salmonella* antibodies was determined with ELISA assay. The suspension of lysates from all strains included in the vaccine was used as the antigen. The blood was collected at 11th and 16th week of life. The mean values obtained for individual groups tested are shown in Fig. 1.

As is apparent from graph shown in Fig. 1, the obtained humoral immune response in chickens vaccinated with Immunovac, was relatively quite low. The immunogenic activity of the vaccine in the 11th week of life was 0.665 OD₄₉₂, and was slightly higher in the 16th week of life (8 weeks post-vaccination) with optical density at ELISA equal to 0.729 OD₄₉₂.

This value is nearly three times lower than the value of the immunogenic activity of the preparation according to the present invention.

### Immunovac vaccine faults

1. The Immunovac consisted of 8 strains of *Salmonella* serotypes which were important from the point of view of epidemiological studies in humans and animals in 1988. Those strains came from the collection of KOS (Krajowy Ośrodek Salmonella), and were obtained from the collection of strains isolated in the 1950s and repeatedly passaged in artificial media.
2. The strains were mixed with native rabbit sera, which were added in excess, because there were no serum proportioning methods for inactivated strains developed, resulting in uncontrolled antibody binding to bacteria, and thus blocking of epitopes - LPS fractions responsible for specific *Salmonella* serotype recognition, in the animal body after vaccination.
3. The determination of bacterium count based on the weight of the resulting bacterial suspension proved to be incorrect. During the production on an industrial scale the centrifugation did not allow to obtain a prescribed concentration of bacteria. Finally, with the product used in breeding flocks, the birds (hens), received much less inactivated bacteria. This resulted in only 80% protection against infection of hens. In studies of vaccinated hen sera, not described in the publication, ELISA assays indicated low levels of antibodies (Fig. 1).
4. The laboratory scale preparation methods used did not correspond to the technology used on large/industrial scale.

For this reason, there is still a strong need in the field for an effective, polyvalent preparation for treatment and/or prevention of salmonelloses in animals, particularly birds.

Therefore, the aim of the invention was to develop a safe and effective preparation for treatment and/or prevention of salmonelloses caused by serotypes from various *Salmonella* strains, especially salmonelloses in animals, particularly birds, which will be easy to manufacture, will neither contain live bacteria, nor require use of specifically mutated strains of bacteria, nor any additional active ingredients such as antibiotics or immunomodulatory substances, in order to obtain the desired effect, and therefore the production thereof will not be complicated, costly or time consuming.

The aim of the invention was to develop a vaccine free of the faults of the known prior art vaccines, especially the faults of the Immunovac vaccine.

The aim of the invention was to develop a vaccine exhibiting a higher efficacy compared to the known vaccines, which when administered does not block epitopes of LPS fractions responsible for recognition of specific *Salmonella* serotypes.

The aim of the invention was also to develop a vaccine that could be readily administered to animals, particularly birds, in particular such one that could be administered in drinking water. Another aim of the invention was to develop a vaccine against salmonelloses that could be administered to young individuals, right from the first day of their life.

Unexpectedly, it was found that the preparation consisting of inactivated *Salmonella* strains of various serotypes, together with antibodies bound thereto, is a safe and effective preparation for the treatment and/or prevention of salmonelloses caused by strains of *Salmonella* belonging to various serotypes, especially in animals. This preparation meets all the objectives of the present invention, and is suitable for the production of a vaccine against salmonelloses, adapted to be administered orally, for example in drinking water, as well as parenterally, and when administered in this manner has a similar or even better efficacy than the vaccine obtained by conventional methods for antisera preparation, particularly suitable for parenteral administration. The purpose of such developed preparation and vaccine is the induction of passive and active immunity against infectious zoonoses and, especially salmonelloses in animals, particularly birds.

Thus, the object of the present invention is a polyvalent, combined immunising and/or therapeutic preparation against salmonellosis, comprising inactivated *Salmonella* sp. strain with antibodies bound thereto, characterized in that the said antibodies
are obtained separately by immunization with antigens derived from *Salmonella* strain having lipopolysaccharide (LPS) containing O-specific polysaccharides (OPS) which are different than O-specific polysaccharides (OPS) of lipopolysaccharide (LPS) of said inactivated *Salmonella* sp. strain
and **in that** the preparation contains said inactivated *Salmonella* sp. strain and said antibodies, bound to the said inactivated *Salmonella* sp. strain, wherein the antibodies are present in an amount of 25-100 mg per bacterial mass of *Salmonella* in concentration of 2-5x10¹³ MPN (most probable number), wherein the combination of inactivated *Salmonella* sp. strain with antibodies bound thereto is selected from inactivated bacteria of O:4 group bound to anti-O:9 immunoglobulins produced by immunising a host using *Salmonella* from O:9 group, and/or inactivated bacteria of O:9 group bound to anti-O:4 immunoglobulins produced by immunising a host using *Salmonella* from O:4 group, and/or inactivated bacteria of O:6.7 group bound to anti-O:6.8 immunoglobulins produced by immunising a host using *Salmonella* from O:6.8 group, and/or inactivated bacteria of O:6,8 group bound to anti-O:6,7 immunoglobulins produced by immunising a host using *Salmonella* from O:6,7 group, such as inactivated bacteria of *S.* Enteritidis O:9 bound to immunoglobulins produced by immunising a host using *S. Paratyphi B* O:4, and/or inactivated bacteria of *S.* Typhimurium O:4 bound to immunoglobulins produced by immunising a host using *S.* Kapemba O:9, and/or inactivated bacteria of *S.* Hadar O:6,8 bound to immunoglobulins produced by immunising a host using *S.* Choleraesuis O:6,7, and/or inactivated bacteria of *S.* Virchow O:6,7 bound to immunoglobulins produced by immunising a host using *S.* Newport O:6,8, and/or inactivated bacteria of *S.* Infantis O:6,7 bound to immunoglobulins produced by immunising a host using *S.* Newport O:6,8.

Preferably, said antibodies are obtained after immunization of animals with a suspension of bacteria inactivated by boiling.

Preferably, the preparation of the invention comprises inactivated bacteria of the *Salmonella* genus *enterica* species, subspecies: *enterica, salamae, arizonae,* and/or *diarizonae, houtenae, Indica.*

Preferably, the preparation of the present invention is characterized in that the inactivated bacteria of the *Salmonella* genus belong to least one of the following serogroups:
From 4 (B) group, particularly serovars: *Salmonella* Typhimurium, *Salmonella* Typhimurium (e.g., 1,4, [5], 12: i: -), *Salmonella* Agona, *Salmonella* Bredeney, *Salmonella* Heidelberg, *Salmonella* Indiana, *Salmonella* Saintpaul, *Salmonella* Brandenburg, *Salmonella* Agama, *Salmonella* Derby, *Salmonella* Stanley, *Salmonella* Paratyphi B, *Salmonella* Schleissheim, *Salmonella* Chester;
From 6,7 and 8,20 (C1 - C3) group, particularly serovars: *Salmonella* Paratyphi C, *Salmonella* Infantis, *Salmonella* Hadar, *Salmonella* Mbandaka, *Salmonella* Livingstone, *Salmonella* Virchow, *Salmonella* Ohio, *Salmonella* Montevideo, *Salmonella* Tennessee, *Salmonella* Rissen, *Salmonella* Bareilly, *Salmonella* Oranienburg, *Salmonella* Thompson, *Salmonella* Newport, *Salmonella* Kottbus, *Salmonella* Kentucky, *Salmonella* Albany, *Salmonella* Bovismorbificans, *Salmonella* Djugu, *Salmonella* Breanderup, *Salmonella* Jeruzalem, *Salmonella* Chester, *Salmonella* Goldcoast, *Salmonella* Braenderup;
From 9 and 9,46 (D1 - D2) group, particularly serovars: *Salmonella* Enteritidis, *Salmonella* Panama, *Salmonella* Dublin, *Salmonella* Gallinarum, *Salmonella* Miami, *Salmonella* Sendai, *Salmonella* Wernigerode, *Salmonella* Typhi;
From 3,10[15, 34] and 1,3,19 (E1 - E4) group, particularly serovars: *Salmonella* Give, *Salmonella* Anatum, *Salmonella* London, *Salmonella* Meleagridis, *Salmonella* Cambridge, *Salmonella* Minneapolis, *Salmonella* Simsbury, *Salmonella* Senftenberg, *Salmonella* Westerstede;
From 11 (F) group, particularly *Salmonella* Aberdeen;
From 13 (G1 - G2) group, particularly serovars: *Salmonella* Cubana, *Salmonella* Poona;
From 6,14 (H) group, particularly serovars: *Salmonella* Heves, *Salmonella* Onderstepoort;
From 16 (I) group, particularly serovars: *Salmonella* Brazil, *Salmonella* Hvittingfoss;
From 17 (J) group, particularly serovar *Salmonella* Berlin;
From 18 (K) group, particularly serovar *Salmonella* IIIa (O:18);
and from further serogroups numbered 21, 28, 30, 35, 38, 39, 40, 41, 42, 43, 44, 45, 47, 48 and 50.

Preferably, the preparation of the present invention is characterized in that the said antibodies are isolated from serum of a rabbit immunised with *Salmonella* antigens.

Preferably, the preparation of the present invention is characterized in that the said antibodies are isolated from the egg yolk of a bird, in particular hen, immunised with *Salmonella* antigens.

Preferably, the preparation of the present invention is characterized in that it contains antibodies isolated from serum of a rabbit immunised with *Salmonella* antigens and the antibodies isolated from the egg yolk of a bird, in particular hen, immunised with *Salmonella* antigens.

Preferably, the preparation of the present invention is characterized in that it contains antibodies in an amount of 50 mg/2-5x10¹³ of bacteria.

Preferably, the preparation of the present invention is for use as a pharmaceutical composition, in particular as a vaccine.

Preferably, the preparation of the present invention is for use in the immunisation and/or treatment of salmonelloses in animals, particularly birds.

The present invention also relates to a method for production of the polyvalent, combined immunising and/or therapeutic preparation, characterized in that it comprises the steps where:
- Antibodies are obtained separately by immunising a host with selected *Salmonella* antigen;
- A sterile bacterial suspension is prepared separately, from the selected bacterial strain of *Salmonella;*
- To each sterile bacterial suspension prepared, obtained antibodies are added to provide a spontaneous binding of antibodies to non-specific O-polysaccharide fractions;
- Inactivated bacteria with antibodies adsorbed thereto are mixed together;
- The obtained suspension is centrifuged and the resulting pellet is resuspended in a physiological saline, optionally with the addition of a preservative, preferably formaldehyde or phenol, or a mixture thereof, at a concentration of not more than 0.05%;
- The obtained preparation with antibodies absorbed on *Salmonella* bacteria is isolated from the reaction medium by centrifugation, wherein in step c) inactivated bacteria of O:4 group are mixed with and bound to anti-O:9 immunoglobulins produced by immunising a host using *Salmonella* from O:9 group, and/or inactivated bacteria of O:9 group are mixed with and bound to anti-O:4 immunoglobulins produced by immunising a host using *Salmonella* from O:4 group, and/or inactivated bacteria of O:6.7 group are mixed with and bound to anti-O:6.8 immunoglobulins produced by immunising a host using *Salmonella* from O:6.8 group, and/or inactivated bacteria of O:6,8 group are mixed with and bound to anti-O:6,7 immunoglobulins produced by immunising a host using *Salmonella* from O:6,7 group, such as inactivated bacteria of *S. Enteritidis* O:9 are mixed with and bound to immunoglobulins produced by immunising a host using *S. Paratyphi B* O:4, and/or inactivated bacteria of *S. Typhimurium* O:4 are mixed with and bound to immunoglobulins produced by immunising a host using *S. Kapemba* O:9, and/or inactivated bacteria of *S. Hadar* O:6,8 are mixed with and bound to immunoglobulins produced by immunising a host using *S*. *Choleraesuis* O:6,7, and/or inactivated bacteria of *S*. *Virchow* O:6,7 are mixed with and bound to immunoglobulins produced by immunising a host using *S. Newport* O:6,8, and/or inactivated bacteria of *S. Infantis* 0:6,7 are mixed with and bound to immunoglobulins produced by immunising a host using *S. Newport* O:6,8.

Preferably, the method of the present invention is characterized in that the sterile bacterial mass is mixed with the antibodies obtained from sera of rabbits immunised with antigens derived from *Salmonella.*

Preferably, the method of the present invention is characterized in that the sterile bacterial mass is mixed with the antibodies derived from egg yolk of birds immunised with the antigens derived from *Salmonella.*

Preferably, the method of the present invention is characterized in that the sterile bacterial mass is mixed with the antibodies obtained from sera of rabbits and egg yolk of birds immunised with antigens derived from *Salmonella.*

Preferably, the method of the present invention is characterized in that the concentration of bacteria in the preparation is not less than 10¹² bacteria/ml and not more than 10¹⁴ bacteria/ml.

The present invention relates to the use of the preparation of the invention for production of a vaccine against salmonelloses in animals, particularly birds.

Another object of the present invention is an anti-salmonellosis vaccine characterized in that it comprises a preparation of the invention and a pharmaceutically acceptable carrier.

Preferably, the vaccine of the present invention is characterized in that it is for use in the immunisation and/or treatment of salmonelloses in animals, particularly birds, which is intended for administration in drinking water.

Preferably, the vaccine of the present invention is characterized in that it is intended for administration to young individuals from the first day of their life.

Preferably, the vaccine of the present invention is characterized in that its administration is repeated 3 or 4 times, preferably every 7 to 10 days.

### Description of the drawings

Fig. 1 The level of anti*-Salmonella* antibodies in sera of hens vaccinated with Immunovac vaccine of the prior art;
Fig. 2 The level of anti*-Salmonella:* Enteritidis, Typhimurium, Hadar, Virchow and Infantis antibodies in sera of hens vaccinated 4 times with preparation of Example Ila;
Fig. 3 The level of anti*-Salmonella:* Enteritidis, Typhimurium, Hadar, Virchow and Infantis antibodies in eggs of hens vaccinated 4 times with preparation of Example Ila;
Fig. 4 The level of anti-SE antibodies in eggs of hens vaccinated with live attenuated *Salmonella* strain *per os* and Selenvac T vaccine given in an injection.

In the following years, the inventors conducted intensive research to clarify the causes of Immunovac vaccine faults. In addition, it was decided to explore the scientific basis and new possibilities of the preparation that had to have protective and therapeutic properties against salmonelloses.

The vaccines of the present invention comprise several strains (in particular 1 to 7) of various serotypes. Bacteria are grown in an enriched agar medium (1.5-2%) and inactivated (killed) with formaldehyde.

Extremely valuable feature of vaccine of the invention is that its composition has been developed based on the complicated chemical structure of the somatic antigen - lipopolysaccharide (LPS) - an endotoxin, based on the conducted studies of the chemical structure of selected fractions of O-specific polysaccharides (OPSs).

LPS is located in the external part of the cell wall of Gram-negative bacteria. It consists of lipid A, KDO (keto-deoxyoctulosonate) - core oligosaccharide and OPSs. Lipid A is an important pathogenic factor, largely responsible for pyrogenic reactions of the higher organisms to infection. The chemical structure of lipid A and KDO is similar in all Gram-negative strains. An OPS is located outside the cell wall. An OPS, showing strong antigenic properties, constitutes the most peripheral part of the LPS molecule. It is composed of repeating oligosaccharide units, up to several dozens of units in each chain, which form a linear structure. Each repeating unit is composed mostly of 2-8 saccharide residues. An OPS is characterized by a very large variation in the saccharide composition, the type of bonds and non-saccharide substituents, both within the genus and species of bacteria. These characteristics are the basis of serological diversity of *Salmonella.*

The most common monosaccharides in an OPS are glucose (Glc) and galactose (Gal), often mannose (Man), ribose, arabinose and xylose, 6-deoxy saccharides (rhamnose (Rha), and fucose), 3,6-dideoxy saccharides (abequose (Abe), tyvelose (Tyv) - 3,6-dideoxy-D-xylohexose).

To define the primary structures of O-specific polysaccharides of selected *Salmonella* bacteria some test were performed using chemical, spectroscopic, enzymatic and immunological methods. Chemical tests included:
- Determination of the saccharide composition;
- Assignation of D- or L-configuration to the saccharides;
- Determination of the binding mode of the saccharide residues;
- Determination of the saccharide component ring sizes;
- Determination of the saccharide residue sequence;
- Determination of the configuration of monosaccharide anomeric carbon atoms;
- Identification of saccharide components, their attachment site and stereochemical characteristics.

Below are examples of structures of repeating O-specific units in the basic *Salmonella* groups that were the basis for the development of theoretical assumptions and subsequently used in the composition of the vaccine.

### O:4 (1,4,12) group

α-Abep                                        α-D-Glcp
↓3                                                ↓4
→2)-α-D-Manp(1→4)-α-L-Rhap-(1→3)-α-D-Galp-(1→

### O:7 (6,7,14) group

α-D-Glcp

↓3

→2)-β-D-Manp(1→2)-α-D-Manp(1→2)-α-D-Manp(1→2)-β-D-Manp(1→3)-β-D-GlcpNAc -(1→

### O:8 (6,8,20) group

α-Abep                                        2-O-Ac-α-DGlcp
↓3                                                ↓3
→4)-β-L-Rhap-(1→2)-α-D-Manp(1→2)-α-D-Manp(1→3)-β-D-Galp-(1→
↑2
O-Ac

### O:9 (1, 9.12) group

α-Tyvp                                        α-D-Glcp
↓3                                                ↓4
→2)-α-D-Manp(1→4)-α-L-Rhap-(1→3)-α-D-Galp-(1→

### O:9,46 (3,9,46) group

α-Tyvp                                        α-D-Glcp
↓3                                                ↓4
→6)-β-D-Manp(1→4)-α-L-Rhap-(1→3)-α-D-Galp-(1→

### 0:3,10 group

O-Ac
↓6
→6)-β-D-Manp(1→4)-α-L-Rhap-(1→3)-α-D-Galp-(1→

### Immunoglobulins - antibodies

Specific identification and elimination of the huge range of antigens requires a wide range of factors involved in the pathogen control. Recognition of epitopes is possible due to the clonal selection and proliferation of cells expressing receptors on their surface: immunoglobulins on B cells and TCR receptors (T cell receptors) on T cells. Antibodies directly recognize epitopes - T cells react to epitopes bound to histocompatibility antigens on the cell surface. A characteristic feature of effector B cells is the production of specific antibodies. A typical antibody molecule is a tetramer composed of two light chains, each of which consists of 220 amino acids, and two heavy chains consisting of 440 amino acids each.

Each of the chains has intrachain disulphide bridges determining their spatial configuration. Saccharide moieties are attached to the heavy chains: the antibody molecule is a glycoprotein. Saccharide moieties, bound to the immunoglobulin molecule, affect the physicochemical properties of these molecules: cause a greater solubility in a polar medium (e.g., water), increase the thermal resistance, protect from digestion by proteases, and maintain the proper conformation of the immunoglobulin molecule. For B cells to be able to transform into plasma cells and produce antibodies there must be a comprehensive response of the immune system.

The decisive moment for starting the chain of reactions is the appearance of the antigen in the body.

Antibodies produced during the primary response belong mainly to the IgM class and their highest level is 7-10 days post-immunization. If case there is no re-immunization, the antibody level decreases, but memory B and T cells remain in circulation. If the antigen is introduced again the body reacts more rapidly and more effectively what is the part of an anamnestic immune response. This is an important principle, which was adopted in our study. The inventors suggest from 3, preferably up to 4 administrations of the vaccine. The amount of antibodies produced is many times greater than during the primary response, and they have greater affinity for the antigen. The secondary response may persist for years (D. Kunikowska, Natural and synthetic antigens of bacteria and viruses, Ann. Acad. Med. Gedan, vol. 36, suppl. 4, 9-141). The dominant antibody class in the secondary response are the antibodies of IgG class. They exhibit a higher affinity for binding to the antigen than the primary response antibodies. The egg yolk antibodies are mainly Igy. Antibodies are an extremely sensitive and excellent tool, they recognize each feature in the structure of polysaccharide antigens: saccharide composition, D- or L-configuration, mode of saccharide residue binding, saccharide component ring size, sequence of the saccharide residues, the configuration of the monosaccharide anomeric carbon atoms and the attachment site of saccharide components.

To construct a new vaccine, immunoglobulins were used from sera obtained from rabbits immunised with properly prepared *Salmonella* antigens. They were isolated and purified by column chromatography using a T-Gel adsorbent and rechromatography on Sephadex G-25. Obtained immunoglobulin suspension contained a mixture of IgG and IgM antibodies. For each batch the protein content was tested by spectrophotometric method, the antibody content by ELISA (enzyme immunoassay), and a quantitative study of specific antibodies using the tube agglutination test was performed. Moreover, isolated Igy antibodies derived from egg yolks of hens vaccinated with appropriate antigens of *Salmonella* were used. The same method of purification as for rabbit immunoglobulins was used. In the control tests the protein level was determined spectrophotometrically and the level of specific antibodies using the tube agglutination test.

In order to learn the basics of the practical assumptions for the constructed vaccine, comprehensive immunological tests of the selected OPSs were performed, which previously have been subjected to fractionation, so that fragments of individual saccharide residues were obtained.

Tests included:
- Preparation of a set of rabbit sera of more than 30 species, which were used to determine the LPS somatic antigens and identify individual fractions of O-specific polysaccharide epitopes;
- Developing several hybrid lines to produce monoclonal antibodies;
- Identification of antigenic epitopes of the individual components of the saccharide residues using ELISA assays, haemagglutination, haemagglutination inhibition and tube agglutination test.

The test results led to conclusions that:
In case of the OPS saccharide residues of O:4 and O:9 groups, both the anti-O:4 and anti-O:9 antibodies reacted with saccharides in the complete basic chains: 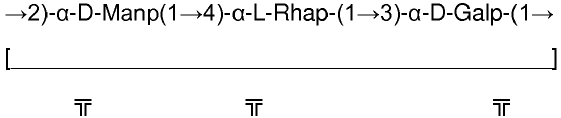
→2)-α-D-Manp(1→4)-α-L-Rhap-(1→3)-α-D-Galp-(1→
[?] [?] [?]
 and with the saccharides in a side chain: 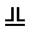 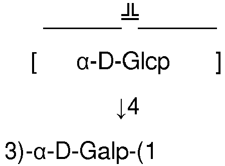
[ α-D-Glcp ]
↓4
3)-α-D-Galp-(1
 and 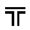 symbols represent the binding site of antibodies.

In order to identify the fragments which are responsible for immunological specificity the rhamnose was oxidized and group O:4 and O:9 epitopes were obtained which remained free, not bound to anti-O:4 and/or anti-O:9 antibodies:
In O:4 group - 
α-Abep
↓3
D-Manp(1→4)-
In O:9 group -

α-Tyvp
↓3
D-Manp(1→4)-

In case of the OPS saccharide residues of O:6 group, anti-O:6 antibodies bind in the following sites:

α-D-Glcp
↓3
→2)-β-D-Manp(1→2)-α-D-Manp(1→2)-α-D-Manp(1→2)-β-D-Manp(1→3)-β-D-GlcpNAc-(1→
[________________________________]
╦ ╦

The sequence of the saccharide residues in the basic chain of these bacteria is mainly composed of mannose linked in (1→2). It is mainly responsible for the induction of anti-O:6 antibody formation. Responsible for antigenic specificity is the fragment:
**2)-β-D-Manp(1→3)-β-D-GlcpNAc-(1**→ the epitope remained free, not bound to antibodies.

In case of the OPS saccharide residues of O:8 group, anti-O:8 antibodies bind in the following sites:

α-Abep                                        2-O-Ac-α-DGlcp
↓3                                                ↓3
→4)-β-L-Rhap-(1→2)-α-D-Manp(1→2)-α-D-Manp(1→3)-β-D-Galp-(1→
↑2
O-Ac

The basic saccharide fragment linking these two groups is the fragment:

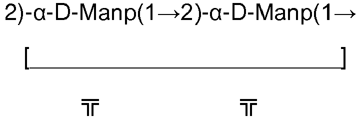
2)-α-D-Manp(1→2)-α-D-Manp(1→

Responsible for the antigenic specificity of this group is the following epitope, which remained free, not bound to antibodies:

α-Abep
↓3
→4)-β-L-Rhap-(1→
↑2
O-Ac

Whereas there is a clear difference between the chemical structure of this group and the O:4 group, where abequose also occurs,
**O:9,46 (3,9,46) group** 
α-Tyvp                                    α-D-Glcp
↓3                                          ↓4
→6)-β-D-Manp(1→4)-α-L-Rhap-(1→3)-α-D-Galp-(1→
 **0:3,10 group**

O-Ac
↓6
→6)-β-D-Manp(1→4)-α-L-Rhap-(1→3)-α-D-Galp-(1→

In the O:9,46 and O:3:10 groups, identical serological reactions indicated common saccharides located in the basic chain:

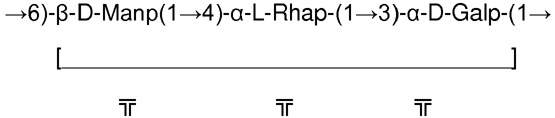
→6)-β-D-Manp(1→4)-α-L-Rhap-(1→3)-α-D-Galp-(1→

Anomeric configuration of β-D-Manp and 1→6 link to galactose make that the antibodies prepared for O:4 and O:9 groups did not react with the these O:9,46 and O:3,10 antigen fractions.

O-specific fraction (epitope) for O:9,46 group is composed of:

α-Tyvp
↓3
D-Manp(1→4)-

This epitope remained free, not bound to antibodies:
and for O:10 group 
O-Ac
↓6
α-D-Galp-(1→
 An O-acetyl is an essential component of the factor 10 antigen determinant.

Immunochemistry schemes presented above are the basis for the development of vaccines of the present invention.

### Development of a new vaccine /formulation/

The above research results led to the development of theoretical bases to produce a new vaccine. In accordance with the described theoretical assumptions a preparation immunizing against salmonelloses was made.

Despite the identical LPS composition, not all serotypes belonging to the shown serogroups have the same virulence. The whole bacteria are responsible for their pathogenic activity. However, the recognition and the immune response depend on the presence of the most important epitopes, factors contained in the O-polysaccharide chain. This is of particular importance in case of re-immunization and in the course of infection.

Immunoglobulin properties described and used in a new vaccine allowed for use antibodies in a vaccine by binding them to specific antigenic epitopes (factors) in the structures of repeating O-specific units in the basic groups of *Salmonella* bacteria, which are responsible for the nature of reaction with the host immune system, without blocking the epitopes responsible for the recognition of *Salmonella* serotype. This feature is particularly important in view of the specific characteristics of these bacteria: "The vaccines made from one serotype of *Salmonella* do not cause cross-immunity to other serotypes" (Meyer H. et al. - "Salmonella and Salmonellosis" Symposium Ploufragan/Saint-Brieuc, France, 1992, p. 345. Segall, T. & Lindberg, A. A. (1993). "Oral vaccination of calves with an aromatic-dependent Salmonella dublin (O9, 12) hybrid expressing O4, 12 protects against S. dublin (O9, 12) but not against Salmonella typhimurium (O4, 5, 12). Infect Immun 61, 1222-1231)."

The conducted studies resulted in development of a new base for technology of anti-salmonellosis vaccine production. Procedures have been developed in accordance with the guidelines of the Polish Pharmacopoeia IX vol. I, 2011 ed.: (Ph. Eu. VII).

The basis constituted *Salmonella* strains. Strains were obtained from the collections of the KOS (National Centre for Salmonella) which were isolated in recent years from people or birds infected with *Salmonella.* The most important bacteria considered were: *Salmonella* Enteritidis (O:9, D₁ group), *Salmonella* Typhimurium (O:4, B group), *Salmonella* Hadar (O:6,8 C₂,₃ group), *Salmonella* Virchow (O:7, C₁ group) and *Salmonella* Infantis (O:7, C₁ group). The subject matter of the studies was also strains of other serogroups. Basic serological and biochemical characteristics of the strains were examined and described. These strains provided a parental seeding batch and were used to develop working stocks. The use of strains passaged in animals (in this case hens) is important because of the loss of some of the features observed after repeated passages on artificial media.

For the production of somatic antigens - O, for the vaccination of animals, a suspension of bacteria inactivated by boiling was used. This treatment allowed removing the flagellar antigens, and left somatic antigens unchanged.

To construct a "new" vaccine, immunoglobulins from sera obtained from rabbits or egg yolks of hens immunised with such antigens were used. Isolated immunoglobulins included antibodies to all components of lipopolysaccharide antigens, as well as to all O-polysaccharide fractions. To obtain a therapeutic function (passive protection of young birds) antibodies were bound to bacteria, which are used for active immunisation. To achieve this goal, antibodies were bound to saccharides in complete basic chains or fragments common for several "somatic" groups described in the Kauffman-White scheme. On the other hand, the epitopes responsible for the basic functions of bacteria recognition by the immune system were left unbound. One can call it an alternate antigen and antibody binding.

And so: Bacteria from O:4 group (e.g., *S.* Typhimurium 1,4,12, *S.* Typhimurium (e.g., 1,4, [5], 12: i: -), *S.* Agona, *S.* Bredeney, *S.* Heidelberg, *S.* Indiana, *S.* Saintpaul, *S.* Brandenburg, S. Derby, *S.* Stanley, *S.* Paratyphi B) were bound to anti-O:9 immunoglobulins, i.e., the ones produced as a result of host immunisation with *Salmonella* from O:9 group.

Bacteria from O:9 group (e.g., *S.* Enteritidis 1,9,12, S. Panama, *S.* Dublin, *S.* Gallinarum) were bound to anti-O:4 immunoglobulins, i.e., the ones produced as a result of host immunisation with *Salmonella* from O:4 group.

Bacteria from 0:6,7 group (e.g., *S.* Infantis 6,7, *S.* Virchow 6,7, and *S.* Mbandaka, *S.* Livingstone, *S.* Tennessee, *S.* Bareilly, *S.* Oranienburg, *S.* Thompson) were bound to anti-O:6,8 immunoglobulins, i.e., the ones produced as a result of host immunisation with *Salmonella* from O:6,8 group.

Bacteria from O:6,8 group (e.g., *S.* Hadar 6,8, *S.* Newport, *S.* Kottbus, *S.* Kentucky, *S.* Albany, *S.* Bovismorbificans, *S.* Breanderup, *S.* Jeruzalem, *S.* Chester, *S.* Goldcoast) were bound to anti-O:6,7 immunoglobulins, i.e., the ones, produced as a result of host immunisation with *Salmonella* from 0:6,7 group.

The above immunochemical schemes describe the basis for this approach.

The studies found also that another key element, apart from binding inactivated bacteria to specific antibodies that bind to antigenic epitopes (factors) in the structures of repeating O-specific units in the basic groups of *Salmonella,* which are responsible for the nature of the reaction with host immune system and yet not block the epitopes responsible for the recognition of *Salmonella* serotype, is the selection and amount of these antibodies. It turned out that the use of antibodies in excess can also lead to blocking of the epitopes, which as previously stated, should remain free, thereby reducing the efficacy of the vaccine, as was the case with Immunovac vaccine, where more than 10 times higher antibody amount was used.

The basis for the development of a polyvalent, combined immunising and/or therapeutic preparation and a vaccine against salmonelloses of the present invention to be administered orally in the drinking water and parenterally since the first days of life of the animals, is a particular susceptibility of young animals to infections. The birds do not have a fully developed immune system until the third week of life. A vaccine based on a preparation of the present invention is safe for humans, vaccinated animals and the environment. It is easy to administer and offers the ability to provide young individuals with the antibodies in the first days of their life, and then causes the development of an active immunity. Moreover, the need for protection against *Salmonella* infections, is required according to the regulations of the European Union.

The possibility of administration of immunising and/or therapeutic preparation and a vaccine of the present invention in drinking water is a very important advantage, because it significantly simplifies the process of prophylactic/therapeutic vaccination of animals, especially birds. It is known, that vaccines are typically administered in the form of preparations for parenteral administration, particularly for injections, especially for intramuscular and subcutaneous injection, due to the significantly increased efficacy in the case of parenteral administration, for example, in comparison to the oral administration. Unexpectedly, the immunising and/or therapeutic preparation and vaccine of the present invention turned to be extremely effective in the case of oral administration in drinking water. As shown later in Example 3 of the specification, the efficacy study of the administered orally vaccine was conducted according to the invention and compared the efficacy of the vaccine to the one administered parenterally, intravenously. A surprising result was generally the comparable efficacy of vaccines administered orally and intravenously and surprisingly the higher efficacy of oral vaccines against some strains of *Salmonella.*

The vaccine of the present invention is a suspension of the bacteria coated with antibodies in physiological saline, i.e., NaCl 8.5 g/l. This is due to the fact that the electrolytes are needed for antibody binding and stabilisation of the preparation. The vaccine may further contain a preservative, which can be selected from formaldehyde and phenol.

In general, the immunising and/or therapeutic preparation for use in bacterial infections and food poisoning, particularly in salmonelloses, which uses a sterile bacterial mass and mono- and polyclonal antibodies, is obtained in the following manner. Sterile bacterial mass of *Salmonella* is mixed with the antibodies obtained from sera of rabbits and/or egg yolk of birds immunised with antigens of *Salmonella,* the whole is left for several hours at preferably 37°C, then centrifuged and the resulting pellet is resuspended in a physiological saline solution, optionally with the addition of a preservative, preferably formaldehyde or phenol, or mixture thereof, having a concentration not exceeding 0.02%. The resulting preparation of antibodies adsorbed on *Salmonella* is isolated from the reaction medium by centrifugation and the concentration of bacteria in the preparation is not less than 10¹² bacteria/ml and no more than 10¹⁴ bacteria/ml.

In the method of the invention, antibodies are preferably isolated from the sera of rabbits immunised with antigens of *Salmonella enterica,* subspecies *enterica,* whereas for this purpose may be used antigens derived from: *Salmonella* Paratyphi B, *Salmonella* Typhimurium, *Salmonella* Heidelberg, *Salmonella* Thompson, *Salmonella* Virchow, *Salmonella* Newport, *Salmonella* Hadar, *Salmonella* Kentucky, *Salmonella* Bovismorbificans, *Salmonella* Kapemba, *Salmonella* Enteritidis, *Salmonella* London, *Salmonella* and *Salmonella* Anatum and/or antibodies obtained from egg yolks of hens immunised with antigens in amounts from 25-100 mg per bacterial mass of *Salmonella* in concentration of 2-5x10¹³ MPN, preferably 50 mg/2-5x10¹³ of bacteria.

The method for production of immunising and/or therapeutic preparation for bacterial infections and food poisoning, particularly by salmonelloses, is characterized by the following examples.

For each step of vaccine production, standard operating procedures were developed.

### Antibody production

Preparation methods for anti*-Salmonella* sera for use in a vaccine according to the present invention, containing antibodies to group and individual factors of somatic antigens, were in accordance with the WHO Collaborating Centre for Reference and Research on *Salmonella* guidelines (M.Y. Popoff, Guidelines for the preparation of Salmonella antisera, 2001, 6th edition, Institut Pasteur, France).

The strains come from the KOS collection. Each of them has a sign, number and specific antigenic patterns. After propagation, the strains become own museum repository.

### Preparing antigens for vaccination

Bacteria - each strain is collected separately, one copy each from own museum repository, inoculated into enriched broth, incubated at 37 C for 18 hours and cultured to obtain single colonies. Carefully selected smooth colonies are passaged on a 1.5% agar medium and a plate with Sven Gard Agar. After 18-hour incubation, the serological identification is performed with a set of slide agglutination sera for *Salmonella* bacteria. In case of strain contamination is noticed, a separate culture is established on a differentiating medium and additional test is performed using a biochemical reaction. Biochemical and serological reactions must comply with the requirements of the WHO Reference Centre.

### Preparation of somatic antigens O for animal vaccination

A single smooth colony is incubated in a broth for 4 hours at 37 C and then transferred on plates with 1.5% agar and cultured at 37 C for 18 hours. The bacteria are washed off with 0.85% NaCl and boiled at 100 C for 2.0 hours in order to destroy the flagelli. After cooling, the sterility of the suspension is verified: 0.1 ml of the suspension is added to 10 ml of broth and incubated for 18 hours at 37 C. No turbidity indicates the sterility of the antigen. In the case the turbidity is noted, the suspension is reboiled for 1 hour at 100 C and the test for sterility is repeated.

Then, the antigen identity check is carried out by tube agglutination test with the standard serum of known antibody titre. Tests are carried out in such a way that a standard serum is serially diluted from 1:10 to 1:1280 and 1-2 drops of the test antigen are added so that the concentration of bacteria is 1.5x10⁸ cfu (Mac Farland scale). The suspension is left overnight at room temperature and the degree of agglutination is recorded on a scale of 1-5. For the vaccination an antigen is selected that exhibits agglutination 5 in a serum dilution of at least 1:640. In this form, the suspension is stable and can be stored for up to 1 year at 2 to 8 C.

The scale for tube agglutination test:
5 - A complete agglutination of bacteria in a completely transparent liquid. Slight tube shake raises cloud of agglutinated bacteria.
4 - A clear agglutination of bacteria in a slightly opalescent liquid.
3 - Bacterial agglomerates in an opalescent liquid.
2 - Slightly visible bacterial agglomerates in an opalescent liquid.
1 - Trace of agglutination in an opalescent liquid.

- indicates no agglutination.

The control is a suspension of bacteria in NaCl.

### Preparation of rabbit antibodies

For the vaccination the antigen is diluted in 0.85% NaCl to a concentration of 2x10⁹ cfu of bacteria (Mac Farland scale). Not purebred rabbits weighing about 2.5 kg are vaccinated. Rabbits are vaccinated first subcutaneously - 0.5 ml of the suspension, and then intravenously with doses of 0.5, 1.0, 2.0 and 4.0 ml every five days. In 9 days after the last vaccination, blood is collected aseptically, allowed to stand for 0.5 hour in an incubator at 37 C, then transferred to a refrigerator (at ±4°C) for 18 hours. After centrifuging the clot (3500 rpm), the serum is transferred into a sterile bottle.

The preliminary sera control is carried out in the tube agglutination test. The serum is serially diluted from 1:10 to 1:2560 and the tests are performed as above (in the description of the antigen identity control).

The test must be performed using a large spectrum of antigens. The selection of antigen depends on the expected co-agglutination and known antigenic factors of the strain used for rabbit vaccination.

Immunoglobulins are isolated from sera by chromatography on a column with T-Gel adsorbent, and rechromatography on a column with Sephadex G-25. After column passage immunoglobulins include IgG and IgM antibodies. They are suspended in 0.85% NaCl, the degree of purification is 96%, and their content is from 13.5 to 20.0 mg/ml.

### Preparation of antibodies from egg yolk

Preparation of anti*-Salmonella* antibodies isolated from chicken egg yolk, for a vaccine to control salmonellosis in hens, is performed based on the guidelines for the production of rabbit anti*-Salmonella* sera. WHO Collaborating Centre for Reference and Research on Salmonella, M.Y. Popoff, Guidelines for the preparation of Salmonella antisera, 2001, 6th edition, Institut Pasteur, France.

Descriptions of the antigen production for vaccination and their controls are the same as for the rabbit sera.

For the vaccination, the antigen is diluted in 0.85% NaCl to a concentration of 2x10⁹ cfu of bacteria (Mac Farland scale). Vaccinated are hens purchased from a certified breeding farm and bred at least 5 weeks before the laying period. Hens are vaccinated first subcutaneously - 0.5 ml of the suspension, and then intramuscularly with doses of 0.5, 1.0, 2.0 and 4.0 ml every five days. Eggs are collected for 3-4 weeks from beginning of the egg laying period. From the entire pool of collected eggs yolks are collected and their antibodies are extracted by ammonium sulphate precipitation, and polyethylene glycol. After dialysis, the crude extract of Igy antibodies is purified by chromatography using T-Gel adsorbent, and then subjected to rechromatography on a column with Sephadex G-25. Igy antibodies obtained from passage through the column are suspended in 0.85% NaCl, 96% purified, and their level was between 200-400 mg/ml.

Based on WHO Collaborating Centre for Reference and Research on *Salmonella* guidelines following exemplary sera are obtained:
anti-O:4 is obtained by vaccination with S. Paratyphi B-4,12 antigen;
anti-O:9 - with S. Kapemba 9,12 antigen;
anti-O:6.7 - with S. Choleraesuis 6,7 antigen;
anti-O:6.8 - with S. Newport 6,8 antigen;

### The basic steps for vaccine preparation

### 1. Preparation of the starting strains

Each strain collected from the working stock batch was cultured and for further work only smooth colonies were selected. Such colonies ensure the full development of the somatic "O" antigen, and this feature is extremely important for efficacy of the preparation. The selected colonies were tested serologically, by slide agglutination test with a set of sera for serological diagnosis of *Salmonella* and tested biochemically with Bio Marieu assays.

### 2. Preparation of the culture broth

Thus prepared strain was inoculated into an enriched broth medium composed of: 1000 ml of distilled water, 0.4 g of meat extract, 5.4 g casein acid hydrolysate, 1.7 g of yeast extract, 3.5 g of sodium hydrochloride, and incubated for 20-24 hours at 37 C.

### 3. Preparation of the sterile bacterial suspension

The bacterial mass was prepared from the selected strain of *Salmonella* in such a manner that on Petri dishes and/or in Roux bottles with a medium composed of: 1000 ml of distilled water, 0.4 g of meat extract, 5.4 g of acid hydrolysate of casein, 1.7 g of yeast extract, 3.5 g of sodium chloride and 15 g of agar, hereinafter referred to as an enriched agar, *Salmonella* bacteria were grown for 24 hours at 37°C. The bacteria were washed off with a physiological saline, formaldehyde was added to a concentration from 0.4% to 0.5% and left at 37°C overnight in order to kill the bacteria. Sterility control of the suspension was performed in accordance with guidelines of the Polish Pharmacopoeia IX vol. I 2011 ed., (Ph. Eu. VII). Bacteria were centrifuged for 60 minutes at 8000-10.000 rpm and the supernatant was discarded. Centrifugation time depends on the volume of the resulting bacterial suspension. The pellet was diluted with physiological saline (NaCl 8.5 g/l, pH about 7.0). The concentration of bacteria was determined using a spectrophotometer at a wavelength of 650 nm. McFarland scale was used as a standard. 4-8x10¹³ MPN (MPN - most probable number) of bacteria was obtained.

### 4. Preparation of the preparation of the invention

For each strain separately, a sterile bacterial mass prepared, as in step 3, containing about 10¹³ MPN, was suspended in 0.85% NaCl solution and added few millilitres of the purified antibodies for the appropriate strain and placed in a water bath with gentle shaking for about 20 hours at about 37 C. Under these conditions, there is a spontaneous antibody binding with nonspecific O-polysaccharide fractions. The suspension was then centrifuged at a rate of 8000-10,000 rpm, the supernatant discarded, and the pellet resuspended in a saline solution, pH of about 7, comprising about 0.02% formaldehyde, resulting in 1000 ml of a suspension containing, in 1 ml approximately 10¹¹ of bacteria. The number of bacteria was determined spectrophotometrically with respect to the McFarland scale, and antibody level were determined with ELISA assay.

Then, in order to formulate the vaccine, from 2 to 7 so separately prepared inactivated bacterial suspensions with antibodies adsorbed thereon, were combined in an appropriate ratio, taking into account the suitability of the vaccine in a specific medium.

### Examples

### Example la

Bacterial mass of S. Enteritidis, prepared as in step 3, containing 2x10¹³ MPN, was suspended in 10 ml NaCl 0,85%, 3 ml of the purified anti-O:4 antibodies (55 mg) was added and placed in a water bath with gentle shaking for about 20 hours at about 37 C. Under these conditions, there is a spontaneous antibody binding with nonspecific O-polysaccharide fractions. In this case it is the basic polysaccharide chain 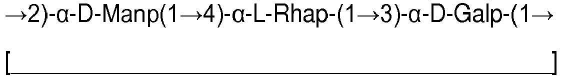
→2)-α-D-Manp(1→4)-α-L-Rhap-(1→3)-α-D-Galp-(1→
 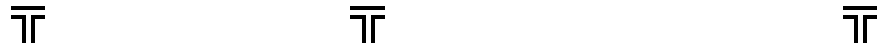 and saccharides in a side chain: 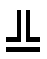 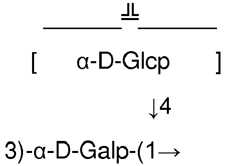
[ α-D-Glcp ]
↓4
3)-α-D-Galp-(1→
 and 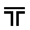 symbols represent the binding site of antibodies

Antigenic epitope responsible for the antigen specificity of bacteria: 
α-Tyvp
↓3
D-Manp(1→4)
 remained free - unbound to antibodies.

The suspension was centrifuged for 60 minutes at a rate of 8000-10,000 rpm, the supernatant discarded, and the pellet resuspended in a saline solution, pH of about 7, comprising 0.02% formaldehyde, resulting in 1000 ml of a suspension containing, in 1 ml 2x10¹¹ of bacteria. The number of bacteria was determined spectrophotometrically with respect to the McFarland scale, obtaining 95% of the theoretical yield. The level of antibodies determined with ELISA assay was about 1.600 for OD₄₉₂.

### Example Ib

Bacterial mass of S. Typhimurium, prepared as in the Example la, containing 1x10¹³ MPN was suspended in 10 ml NaCl 0,85%, 1.5 ml of the purified anti-O:9 antibodies (25 mg) added and placed in a water bath with gentle shaking for 20 hours at 37 C. Antibodies bound to nonspecific O-polysaccharide fractions. In this case, the basic polysaccharide chain is the same as the one of S. Enteritidis: 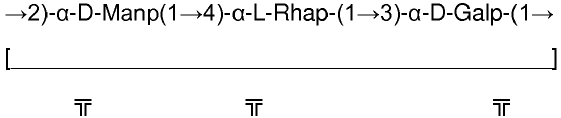
→2)-α-D-Manp(1→4)-α-L-Rhap-(1→3)-α-D-Galp-(1→
[?] [?] [?]
 and saccharides in a side chain:

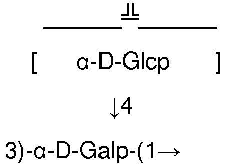
[ α-D-Glcp ]
↓4
3)-α-D-Galp-(1→

Epitope responsible for the antigen specificity of bacteria: 
α-Abep
↓3
D-Manp(1→4)-
 remained free - unbound to antibodies.

The suspension was then centrifuged for 60 minutes at a rate of 8000-10,000 rpm, the supernatant discarded, and the pellet resuspended in a saline solution, pH of about 7, comprising 0.02% formaldehyde, resulting in 1000 ml of a suspension containing, in 1 ml 1x10¹¹ of bacteria. The number of bacteria was determined spectrophotometrically with respect to the McFarland scale, obtaining 98% of the theoretical yield. The level of antibodies determined with ELISA assay was about 1.600 for OD₄₉₂.

### Example Ic

Bacterial mass of S. Hadar, prepared as in the Example la, containing 1x10¹³ MPN was suspended in 10 ml NaCl 0,85%, 1.5 ml of the purified anti-O:6.7 antibodies (25 mg) added and placed in a water bath with gentle shaking for 20 hours at 37 C. Antibodies bound to nonspecific O-polysaccharide fractions.

→2)-α-D-Manp(1→2)-α-D-Manp(1→

[______________________________]

₸ ₸

Epitope responsible for the antigen specificity of bacteria:

α-Abep
↓3
→4)-β-L-Rhap-(1→
↑2
O-Ac remained free – unbound to antibodies.

The suspension was centrifuged for 60 minutes at a rate of 8000-10,000 rpm, the supernatant discarded, and the pellet resuspended in a saline solution, pH of about 7, comprising 0.02% formaldehyde, resulting in 1000 ml of a suspension containing, in 1 ml 1x10¹¹ of bacteria. The number of bacteria was determined spectrophotometrically with respect to the McFarland scale, obtaining 96% of the theoretical yield. The level of antibodies determined with ELISA assay was about 1.600 for OD₄₉₂.

### Example Id

Bacterial mass of S. Infantis, prepared as in the Example la, containing 0.5x10¹³ MPN was suspended in 10 ml NaCl 0,85%, 0.75 ml of the purified anti-O:6.8 antibodies (15 mg) added and placed in a water bath with gentle shaking for 20 hours at 37 C. Antibodies bound to nonspecific O-polysaccharide fractions.

→2)-α-D-Manp(1→2)-α-D-Manp(1→

[______________________________]

₸ ₸

Responsible for antigenic specificity of S. Infantis bacteria is the fragment:
**2)-β-D-Manp(1→3)-β-D-GlcpNAc-(1→** the epitope remained free, not bound to antibodies.

The suspension was centrifuged for 60 minutes at a rate of 8000-10,000 rpm, the supernatant discarded, and the pellet resuspended in a saline solution, pH of about 7, comprising 0.02% formaldehyde, resulting in 1000 ml of a suspension containing, in 1 ml 0.5x10¹¹ of bacteria. The number of bacteria was determined spectrophotometrically with respect to the McFarland scale, obtaining 95% of the theoretical yield. The level of antibodies determined with ELISA assay was about 1.600 for OD₄₉₂.

### Example le

Bacterial mass of S. Enteritidis, prepared as in step 3, containing 2x10¹³ MPN was suspended in 10 ml NaCl 0,85% and then 3 ml of the purified anti-O:4 antibodies (55 mg) isolated from chicken egg yolks was added and placed in a water bath with gentle shaking for about 20 hours at about 37 C. Under these conditions, there is a spontaneous antibody binding with nonspecific O-polysaccharide fractions. In this case it is the basic polysaccharide chain 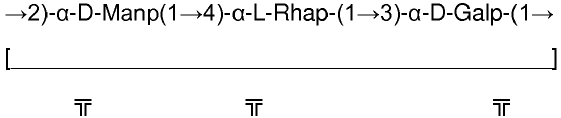
→2)-α-D-Manp(1→4)-α-L-Rhap-(1→3)-α-D-Galp-(1→
[?] [?] [?]
 and saccharides in a side chain: 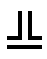 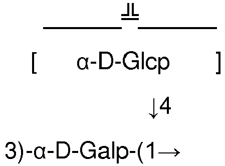
[ α-D-Glcp ]
↓4
3)-α-D-Galp-(1→
 and 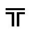 symbols represent the binding site of antibodies.

Antigenic epitope responsible for the antigen specificity of bacteria: 
α-Tyvp
↓3
D-Manp(1→4)
 remained free - unbound to antibodies.

The suspension was centrifuged for 60 minutes at a rate of 8000-10,000 rpm, the supernatant discarded, and the pellet resuspended in a saline solution, pH of about 7, comprising 0.02% formaldehyde, resulting in 1000 ml of a suspension containing, in 1 ml 2x10¹¹ of bacteria. The number of bacteria was determined spectrophotometrically with respect to the McFarland scale, obtaining 95% of the theoretical yield. The level of antibodies determined with ELISA assay was about 1.600 for OD₄₉₂.

### Example If

Bacterial mass of S. Typhimurium, prepared as in the Example la, containing 1x10¹³ MPN was suspended in 10 ml NaCl 0,85% and then 1.5 ml of the purified anti-O:9 antibodies (25 mg) isolated from chicken egg yolks was added and placed in a water bath with gentle shaking for 20 hours at 37 C. Antibodies bound to nonspecific O-polysaccharide fractions. In this case, the primary polysaccharide chain is the same as the one of S. Enteritidis: 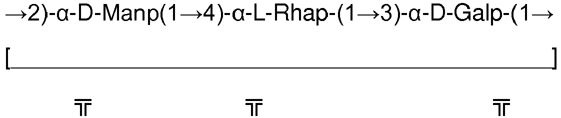
→2)-α-D-Manp(1→4)-α-L-Rhap-(1→3)-α-D-Galp-(1→


 and saccharides in a side chain:

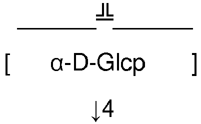
[ α-D-Glcp ]
↓4

3)-α-D-Galp-(1→

Antigenic epitope responsible for the antigen specificity of bacteria: 
α-Abep
↓3
D-Manp(1→4)-
 remained free - unbound to antibodies.

The suspension was centrifuged for 60 minutes at a rate of 8000-10,000 rpm, the supernatant discarded, and the pellet resuspended in a saline solution, pH of about 7, comprising 0.02% formaldehyde, resulting in 1000 ml of a suspension containing, in 1 ml 1x10¹¹ of bacteria. The number of bacteria was determined spectrophotometrically with respect to the McFarland scale, obtaining 98% of the theoretical yield. The level of antibodies determined with ELISA assay was about 1.600 for OD₄₉₂.

### Example Ig

Bacterial mass of S. Hadar, prepared as in the Example la, containing 1x10¹³ MPN was suspended in 10 ml NaCl 0,85% and then 1.5 ml of the purified anti-O:6.7 antibodies (25 mg) isolated from chicken egg yolks was added and placed in a water bath with gentle shaking for 20 hours at 37 C. Antibodies bound to nonspecific O-polysaccharide fractions.

→2)-α-D-Manp(1→2)-α-D-Manp(1→

[________________________________]

₸ ₸

Antigenic epitope responsible for the antigen specificity of bacteria:

α-Abep
↓3
→4)-β-L-Rhap-(1→
↑2
O-Ac remained free – unbound to antibodies.

The suspension was centrifuged for 60 minutes at a rate of 8000-10,000 rpm, the supernatant discarded, and the pellet resuspended in a saline solution, pH of about 7, comprising 0.02% formaldehyde, resulting in 1000 ml of a suspension containing, in 1 ml 1x10¹¹ of bacteria. The number of bacteria was determined spectrophotometrically with respect to the McFarland scale, obtaining 96% of the theoretical yield. The level of antibodies determined with ELISA assay was about 1.600 for OD₄₉₂.

### Example Ih

Bacterial mass of S. Infantis, prepared as in the Example la, containing 0.5x10¹³ MPN was suspended in 10 ml NaCl 0,85% and then 0.75 ml of the purified anti-O:6.8 antibodies (15 mg) isolated from chicken egg yolks was added and placed in a water bath with gentle shaking for 20 hours at 37 C. Antibodies bound to nonspecific O-polysaccharide fractions.

→2)-α-D-Manp(1→2)-α-D-Manp(1→
[________________________]

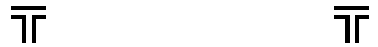

Responsible for antigenic specificity of S. Infantis bacteria is the fragment:
**2)-β-D-Manp(1→3)-β-D-GlcpNAc-(1**→ the epitope remained free, not bound to antibodies.

The suspension was centrifuged for 60 minutes at a rate of 8000-10,000 rpm, the supernatant discarded, and the pellet resuspended in a saline solution, pH of about 7, comprising 0.02% formaldehyde, resulting in 1000 ml of a suspension containing, in 1 ml 0.5x10¹¹ of bacteria. The number of bacteria was determined spectrophotometrically with respect to the McFarland scale, obtaining 95% of the theoretical yield. The level of antibodies determined with ELISA assay was about 1.600 for OD₄₉₂.

### Example IIa

By the method of Examples Ia-Id bacterial masses were prepared separately for each *S.* Enteritidis, *S.* Typhimurium, *S.* Hadar, *S.* Virchow and *S.* Infantis strain, which were combined separately with antibodies isolated from the sera of rabbits vaccinated with bacterial suspensions of: *S.* Paratyphi B O:4, *S.* Kapemba O:9, *S*. Choleraesuis 0:6,7 and *S.* Newport O:6,8, respectively, as shown in the following Table.

| Strain | Antibodies obtained from the serum of rabbits vaccinated with bacterial suspension |
|---|---|
| *S.* Enteritidis O:9 | *S.* Paratyphi B O:4 |
| *S.* Typhimurium B O:4 | *S.* Kapemba O:9 |
| *S.* Hadar O:6,8 | *S.* Choleraesuis O:6,7 |
| *S.* Virchow O:6,7 | *S.* Newport O:6,8 |
| *S.* Infantis O:6,7 | *S.* Newport O:6,8 |

Subsequently, the inactivated bacteria with adsorbed antibodies on them were mixed in the ratio of 2:1:1:0.5:0.5 (based on the amount of bacteria), and the resulting bacterial mass containing 5x10¹³ MPN was suspended in saline solution at pH 7, containing 0.02% formaldehyde, resulting in 1000 ml of a suspension containing 5x10¹¹ bacteria per 1 ml, which constituted 96% of the theoretical yield. The level of antibodies determined with ELISA assay was about 1.600 for OD₄₉₂.

### Example IIb

By the method of Examples Ie-Ih bacterial masses of *S.* Enteritidis, *S.* Typhimurium, *S.* Hadar, *S.* Virchow and *S.* Infantis, bound with antibodies isolated from the egg yolks from hens vaccinated separately with bacterial suspensions of: *S.* Paratyphi B O:4, *S.* Kapemba O:9,*S.* Choleraesuis 0:6,7 and *S.* Newport O:6,8, respectively were prepared separately, (combining method similar to the one in the Table above), in which the suspension of antibodies to combine with the bacteria, as described above, was diluted so as to obtain a concentration corresponding to the concentration of rabbit antibodies. Subsequently, the inactivated bacteria with adsorbed antibodies on them were mixed in the ratio of 2:1:1:0.5:0.5 (based on the amount of bacteria), and the resulting bacterial mass containing 5x10¹³ MPN suspended in saline solution at pH 7, containing 0.02% formaldehyde, resulting in 1000 ml of a suspension containing 5x10¹¹ bacteria per 1 ml, which constituted 96% of the theoretical yield. The level of antibodies determined with ELISA assay was about 1.600 for OD₄₉₂.

### Example III

### The study of antibodies to Salmonella in hens vaccinated with preparation according to Example Ila

Experiments were conducted to determine the specific immune response in hens after administration of a preparation containing 5 inactivated *Salmonella* strains: Enteritidis, Typhimurium, Hadar, Virchow and Infantis (mixed in the following ratios 2:1:1:0,5:0,5), which were bound to antibodies indicated in the Example Ila. The study was conducted on commercial reproductive flocks (laying hens (15,000 animals) and broilers (14,000 animals)). Chickens were vaccinated 4 times, starting from the first week of life, at weekly intervals. The preparation was administered in drinking water: 5,0x10⁷; 1x10^{s}; 2x10⁸ and 4x10⁸/ml. Blood samples were collected from randomly selected animals - from 25 birds in each flock - in the 7th and 17th week of life in case of laying hens (i.e., in the 3rd and 13th week post-vaccination) and in the 7th and 13th week of life in case of broilers (i.e., in the 3rd and 9th week post-vaccination). The level of antibodies was tested with ELISA assay in sera diluted 1:10, and the results are shown in Fig. 2.

### Results and comparison

### Antibody level:

As is apparent from graphs shown in Fig. 1 and Fig. 2 and the Table 1 below, the level of antibodies in the sera of birds vaccinated with the experimental preparation, produced in accordance with Example Ila, was very high for all 5 strains. The graph shows the tendency to antibody level increase after 14th week of life in laying hens. The lowest OD₄₉₂ value was **1.500,** and the highest was **3.000.**

To evaluate the quality of obtained results, one has to refer them to the results of the study of the immune response in laying hens vaccinated four times with Immunovac vaccine, which showed extremely low levels of antibodies with OD₄₉₂ value for laying hens at 11th week of life, equal to **0.665,** and in the 16th week of life - **0.729.**

**Table 1**

| Comparison of antibody levels in sera of birds vaccinated with preparation produced according to Example IIa and Immunovac vaccine | | | | |
|---|---|---|---|---|
| Antigens | Vaccinated birds | | | |
| Preparation of Example Ila | Laying hens | | Broilers | |
| | 7th week | 17th week | 7th week | 13th week |
| *S*. Enteritidis | 1.884* | 2.212 | 2.172 | 1.747 |
| *S*. Typhimurium | 2.301 | 2.811 | 2.026 | 2.485 |
| *S.* Hadar | 2.074 | 3.097 | 2.192 | 2.472 |
| *S.* Virchow | 1.499 | 2.240 | 2.299 | 1.765 |
| *S*. Infantis | 1.893 | 2.570 | 2.547 | 2.221 |
| Negative control | 0.377 | 0.383 | 0.432 | 0.472 |
| | | | | |

| **Immunovac** | | | | |
|---|---|---|---|---|
| Lysate of 8 *Salmonella* strains | 11th week | 16th week | | |
| | 0.665 | 0.729 | | |
| Negative control (chickens in the 2nd week of life) | 0.172 | | | |

| | | | | |
|---|---|---|---|---|
| *OD value at 492 nm | | | | |

### Example IV

### Study of antibody levels in eggs of vaccinated hens

Study of antibody levels in eggs of laying hens vaccinated with a preparation comprising 5 inactivated *Salmonella* strains*:* Enteritidis, Typhimurium, Hadar, Virchow and Infantis (mixed in the following ratios 2:1:1:0,5:0,5), which were bound to antibodies indicated in the Example Ila.

The study was conducted on commercial reproductive flock of 15,000 animals. Chickens were vaccinated 4 times, starting from the first week of life, at weekly intervals. The preparation was administered in drinking water: 5,0x10⁷; 1x10⁸; 2x10⁸ and 4x10⁸/ml. For the study inventors collected randomly 60 eggs from hens at 28th week of their life.

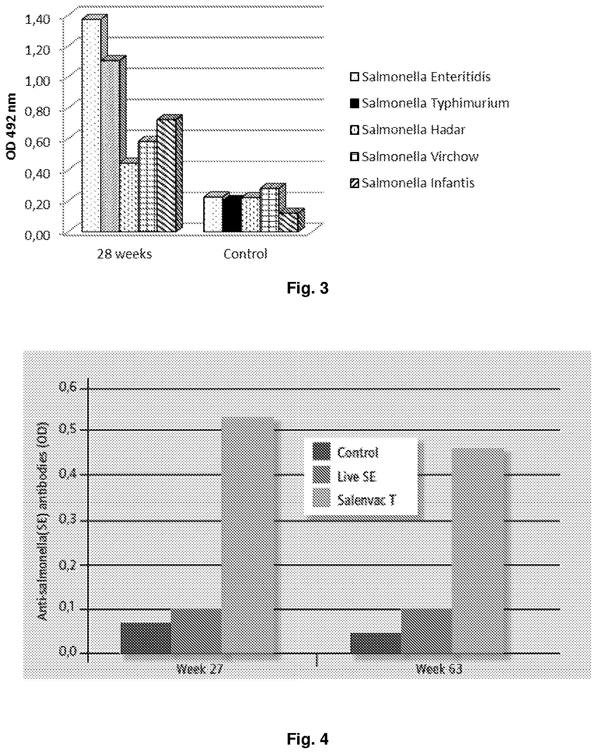

The level of antibodies in a mixture containing egg white and yolk diluted 1:50 was examined with ELISA assay. The results are shown in Fig. 3.

Antibody levels in eggs of vaccinated hens was much higher than in the sera. Optical density values OD₄₉₂ were: for S. Enteritidis - 1.37; S. Typhimurium - 1.10; S. Hadar - 0.44; S. Virchow - 0.58; S. Infantis - 0.72. The results shown are significantly lower than the OD values shown in Fig. 2 (in the hen sera), which is associated with a 40-fold higher dilution of the egg content than the dilution of hen sera.

### Example V

### Comparison of antibody levels after administration of a commercial vaccine and preparation of the invention

Very interesting was the comparison of the results of studies on antibodies contained in the eggs of hens vaccinated with the polyvalent preparation according to the invention with the results of studies performed by Brian Sheehan and Rick van Oort. They conducted a study on antibody levels in eggs of hens vaccinated *per os* with a live attenuated strain (the name of the vaccine was not mentioned in the publication) and Selenvac T vaccine given by injection. The results showed an absence of antibodies in eggs of hens vaccinated with live bacteria whereas the vaccine injection caused the presence of anti-Se (*Salmonella enteritidis*) in the 27th and 63rd week after vaccination (Fig. 4 graph from: Salmonella control: protecting eggs and people. Brian Sheehan and Rick van Oort, World Poultry - vol. 22, no 9. 2006. WP www.World/Poultry. Net 42).

In polyvalent vaccine studies (experimental preparation of Example Ila), after 4 *per os* administrations of a preparation containing inactivated bacteria and antibodies, the inventors found in eggs significantly higher antibody levels to 5 strains contained in the vaccine (Fig. 3), indicating that the vaccine has the ability to protect the progeny of the vaccinated hens.

This finding is extremely important because there no published report found, which would confirm the ability to induce a significant immune response after *per os* application of inactivated bacteria for immunisation. Particularly noteworthy is the fact of the antibody detection in eggs of hens vaccinated with the new preparation. The explanation of this phenomenon may be the reaction of the immune system on repeated vaccine administration.

### Example VI

### Antibody digestion

Studies were performed, which were very important from point of view of the usefulness of the antibody-containing bacterium complex for the functioning of the preparation after administration via the gastrointestinal tract.

Antibodies were isolated from the sera of rabbits immunised with *Salmonella:* O:4 (B), 0:6,7 (C₁) and O:9 (D₁), using ammonium sulphate precipitation. The precipitate was dissolved in water and dialysed against 0.1 M sodium acetate, pH 4.0. The protein content determined spectrophotometrically (λ = 280 nm) was 100 mg/ml.

The digestion was carried out in two steps:

### Step I

Pepsin digestion (with enzyme concentration 1 mg/33 mg protein) at 37 C and pH 4.0 was carried out for 30 minutes. Samples for the study were collected before the digestion (sample 0), after 15 minutes (sample 1) and after 30 minutes (sample 2). The digestion reaction was quenched by addition of 0.1 M Tris-HCI/0.02 M CaCl₂ to a volume of 1/40 and the pH was adjusted to 7.8.

### Step II

Trypsin digestion (1 mg/40 mg protein). Antibodies digested with pepsin, as above, were subjected to trypsin digestion for 19 hours at 37 C and pH 7.8. Samples for the study were taken: after 1 hour (sample 3), 2 hours (sample 4), 3 hours (sample 5), 4 hours (sample 6) and after 19 hours (sample 7). All samples were stored at - 20 C.

Effects of digestion were studied in tube agglutination test.

As is apparent from the data presented in Table 2, pepsin digestion in an acidic environment caused a decrease in antibody titre of 1 to 2 dilutions, while digestion with trypsin for 19 hours did not result in loss of serological activity of the tested sera.

**Table 2**

| Serological activity of rabbit antibodies digested with pepsin and trypsin | | | |
|---|---|---|---|
| Titre (log₁₀) | | | |
| Sample | Bacterial antigens | | |
| number | O:4 (B) | 0:6,7 (C₁) | O:9 (D₁) |
| 0 | 6400 | 6400 | 3200 |

| Step I | | | |
|---|---|---|---|
| 1 | 3200 | 1600 | 1600 |
| 2 | 1600 | 1600 | 800 |

| Step II | | | |
|---|---|---|---|
| 3 | 1600 | 1600 | 800 |
| 4 | 1600 | 1600 | 800 |
| 5 | 1600 | 1600 | 800 |
| 6 | 1600 | 1600 | 800 |
| 7 | 1600 | 1600 | 800 |

During digestion with pepsin in an acid environment, the antibody serological activities were reduced by two orders of dilution. Enzyme digested antibodies, under conditions similar to those in the gastrointestinal tract, did not lose their bacterial antigen binding properties. The conducted studies show that a vaccine consisting of bacteria and antibody composition may be administered orally, without any considerations regarding a complete antibody degradation in the gastrointestinal tract. Vaccines of such a composition may also be used to control other infectious diseases of the gastrointestinal tract.

Conclusions: 4-time *per os* vaccination showed the ability to induce strong humoral immune response detected in the sera of vaccinated chickens and formation of a significant amount of antibodies in the eggs.

## Claims

1. A polyvalent, combined, immunising and/or therapeutic preparation against salmonellosis comprising inactivated *Salmonella* sp. strain with antibodies bound thereto, **characterized in that** said antibodies are obtained separately by immunization with antigens derived from *Salmonella* strain having lipopolysaccharide (LPS) containing O-specific polysaccharides (OPS) which are different than O-specific polysaccharides (OPS) of lipopolysaccharide (LPS) of said inactivated *Salmonella* sp. strain
and **in that** the preparation contains said inactivated *Salmonella* sp. strain and said antibodies, bound to the said inactivated *Salmonella* sp. strain, wherein the antibodies are present in an amount of 25-100 mg per bacterial mass of *Salmonella* in concentration of 2-5x10¹³ MPN (most probable number), wherein the combination of inactivated *Salmonella* sp. strain with antibodies bound thereto is selected from inactivated bacteria of O:4 group bound to anti-O:9 immunoglobulins produced by immunising a host using *Salmonella* from O:9 group, and/or inactivated bacteria of O:9 group bound to anti-O:4 immunoglobulins produced by immunising a host using *Salmonella* from O:4 group, and/or inactivated bacteria of O:6.7 group bound to anti-O:6.8 immunoglobulins produced by immunising a host using *Salmonella* from O:6.8 group, and/or inactivated bacteria of O:6,8 group bound to anti-O:6,7 immunoglobulins produced by immunising a host using *Salmonella* from 0:6,7 group, such as inactivated bacteria of *S.* Enteritidis O:9 bound to immunoglobulins produced by immunising a host using *S. Paratyphi B* O:4, and/or inactivated bacteria of *S.* Typhimurium O:4 bound to immunoglobulins produced by immunising a host using *S.* Kapemba O:9, and/or inactivated bacteria of *S.* Hadar O:6,8 bound to immunoglobulins produced by immunising a host using *S.* Choleraesuis O:6,7, and/or inactivated bacteria of *S.* Virchow 0:6,7 bound to immunoglobulins produced by immunising a host using *S.* Newport O:6,8, and/or inactivated bacteria of *S.* Infantis 0:6,7 bound to immunoglobulins produced by immunising a host using *S.* Newport O:6,8.

2. The preparation according to the claim 1, **characterized in that** said antibodies are obtained after immunization of animals with a suspension of bacteria inactivated by boiling.

3. The preparation according to the claim 1 or 2, **characterized in that** it comprises inactivated bacteria of the *Salmonella* genus *enterica* species, subspecies: *enterica, salamae, arizonae* and/or *diarizonae, houtenae, Indica.*

4. The preparation according to any of claims 1-3, **characterized in that** the inactivated bacteria of the *Salmonella* genus belong to least one of the following serogroups:
From 4 (B) group, particularly serovars: *Salmonella* Typhimurium, *Salmonella* Typhimurium (e.g., 1,4, [5], 12: i: -), *Salmonella* Agona, *Salmonella* Bredeney, *Salmonella* Heidelberg, *Salmonella* Indiana, *Salmonella* Saintpaul, *Salmonella* Brandenburg, *Salmonella* Agama, *Salmonella* Derby, *Salmonella* Stanley, *Salmonella* Paratyphi B, *Salmonella* Schleissheim, *Salmonella* Chester;
From 6,7 and 8,20 (C1 - C3) group, particularly serovars: *Salmonella* Paratyphi C, *Salmonella* Infantis, *Salmonella* Hadar, *Salmonella* Mbandaka, *Salmonella* Livingstone, *Salmonella* Virchow, *Salmonella* Ohio, *Salmonella* Montevideo, *Salmonella* Tennessee, *Salmonella* Rissen, *Salmonella* Bareilly, *Salmonella* Oranienburg, *Salmonella* Thompson, *Salmonella* Newport, *Salmonella* Kottbus, *Salmonella* Kentucky, *Salmonella* Albany, *Salmonella* Bovismorbificans, *Salmonella* Djugu, *Salmonella* Breanderup, *Salmonella* Jeruzalem, *Salmonella* Chester, *Salmonella* Goldcoast, *Salmonella* Braenderup;
From 9 and 9,46 (D1 - D2) group, particularly serovars: *Salmonella* Enteritidis, *Salmonella* Panama, *Salmonella* Dublin, *Salmonella* Gallinarum, *Salmonella* Miami, *Salmonella* Sendai, *Salmonella* Wernigerode, *Salmonella* Typhi;
From 3,10[15, 34] and 1,3,19 (E1 - E4) group, particularly serovars: *Salmonella* Give, *Salmonella* Anatum, *Salmonella* London, *Salmonella* Meleagridis, *Salmonella* Cambridge, *Salmonella* Minneapolis, *Salmonella* Simsbury, *Salmonella* Senftenberg, *Salmonella* Westerstede;
From 11 (F) group, particularly *Salmonella* Aberdeen;
From 13 (G1 - G2) group, particularly serovars: *Salmonella* Cubana, *Salmonella* Poona;
From 6,14 (H) group, particularly serovars: *Salmonella* Heves, *Salmonella* Onderstepoort;
From 16 (I) group, particularly serovars: *Salmonella* Brazil, *Salmonella* Hvittingfoss;
From 17 (J) group, particularly serovar *Salmonella* Berlin;
From 18 (K) group, particularly serovar *Salmonella* IIIa (O:18);
and from further serogroups numbered 21, 28, 30, 35, 38, 39, 40, 41, 42, 43, 44, 45, 47, 48 and 50.

5. The preparation according to any of claims 1-4, **characterized in that** said antibodies are isolated from serum of a rabbit immunised with *Salmonella* antigens.

6. The preparation according to any of claims 1-4, **characterized in that** said antibodies are isolated from bird egg yolk, particularly of a chicken egg, from a bird immunised with *Salmonella antigens.*

7. The preparation according to any of claims 1-4 **characterized in that** it contains antibodies isolated from serum of a rabbit immunised with *Salmonella* antigens and antibodies isolated from egg yolk of a bird, in particular hen, immunised with *Salmonella* antigens.

8. The preparation according to any of the claims 1 to 7, **characterized in that** it contains antibodies in an amount of 50 mg/2-5x10¹³ of bacteria.

9. The preparation according to any of the claims 1 to 8 for use as a pharmaceutical composition, particularly as a vaccine.

10. The preparation according to any of the claims 1 to 8 for use in immunisation and/or treatment of salmonelloses in animals, particularly birds.

11. A method for producing the polyvalent, combined immunizing and/or therapeutic preparation according to any one of claims 1 to 8, **characterized in that** it comprises the steps, where:
a) Antibodies are obtained separately by immunising a host with selected *Salmonella* antigen;
b) A sterile bacterial suspension is prepared separately from a selected bacterial strain of *Salmonella;*
c) To each sterile bacterial suspension prepared in step b), antibodies obtained in step a) are added, wherein the antibodies from step a) were obtained by immunization with antigens derived from *Salmonella* strain having lipopolysaccharide (LPS) containing O-specific polysaccharides (OPS) which are different than O-specific polysaccharides (OPS) of lipopolysaccharide (LPS) of the bacteria of said sterile bacterial suspension from step b), to provide a spontaneous binding of antibodies to non-specific O-polysaccharide fractions, and wherein the antibodies are added in an amount of 25-100 mg per bacterial mass of *Salmonella* in concentration of 2-5x10¹³ MPN, preferably 50 mg/2-5x10¹³ of bacteria;
d) Inactivated bacteria with antibodies adsorbed thereto are mixed together;
e) The obtained suspension is centrifuged and the resulting pellet is resuspended in a physiological saline, optionally with the addition of a preservative, preferably formaldehyde or phenol, or a mixture thereof, at a concentration of not more than 0.05%;
f) The obtained preparation with antibodies absorbed on *Salmonella* bacteria is isolated from the reaction medium by centrifugation, wherein in step c) inactivated bacteria of O:4 group are mixed with and bound to anti-O:9 immunoglobulins produced by immunising a host using *Salmonella* from O:9 group, and/or inactivated bacteria of O:9 group are mixed with and bound to anti-O:4 immunoglobulins produced by immunising a host using *Salmonella* from O:4 group, and/or inactivated bacteria of O:6.7 group are mixed with and bound to anti-O:6.8 immunoglobulins produced by immunising a host using *Salmonella* from O:6.8 group, and/or inactivated bacteria of O:6,8 group are mixed with and bound to anti-O:6,7 immunoglobulins produced by immunising a host using *Salmonella* from 0:6,7 group, such as inactivated bacteria of *S. Enteritidis* O:9 are mixed with and bound to immunoglobulins produced by immunising a host using *S. Paratyphi B* O:4, and/or inactivated bacteria of *S*. *Typhimurium* O:4 are mixed with and bound to immunoglobulins produced by immunising a host using S. *Kapemba* O:9, and/or inactivated bacteria of *S*. *Hadar* O:6,8 are mixed with and bound to immunoglobulins produced by immunising a host using *S*. *Choleraesuis* O:6,7, and/or inactivated bacteria of *S*. *Virchow* 0:6,7 are mixed with and bound to immunoglobulins produced by immunising a host using *S. Newport* O:6,8, and/or inactivated bacteria of *S. Infantis* 0:6,7 are mixed with and bound to immunoglobulins produced by immunising a host using *S. Newport* O:6,8.

12. The method according to claim 11 **characterized in that** the sterile bacterial mass is mixed with antibodies obtained from sera of rabbits immunised with antigens derived from *Salmonella.*

13. The method according to claim 11 **characterized in that** the sterile bacterial mass is mixed with antibodies derived from egg yolk of birds immunised with antigens derived from *Salmonella.*

14. The method according to claim 11, **characterized in that** the sterile bacterial mass is mixed with antibodies obtained from sera of rabbits and egg yolk of birds immunised with antigens derived from *Salmonella.*

15. The method according to any of the claims 11 to 14 **characterized in that** the concentration of bacteria in the preparation is not less than 10¹² bacteria/ml and not more than 10¹⁴ bacteria/ml.

16. Use of the preparation according to the claim 1 to 8 for the production of a vaccine against salmonelloses in animals, particularly birds.

17. An anti-salmonellosis vaccine, **characterized in that** it comprises a preparation according to any one of claims 1 to 8 and a pharmaceutically acceptable carrier.

18. The vaccine according to the claim 17 for use in the immunisation and/or treatment of salmonelloses in animals, particularly birds, which is intended for administration in drinking water.

19. The vaccine for use according to the claim 18, which is intended for administration to young individuals from the first day of their life.

20. The vaccine for use according to the claim 18 or 19 which administration is repeated 3 or 4 times, preferably every 7 to 10 days.

## Patentansprüche

1. Mehrwertiges, kombiniertes, immunisierendes und/oder therapeutisches Präparat gegen Salmonellose, umfassend einen inaktivierten Salmonella sp.-Stamm mit daran gebundenen Antikörpern, **dadurch gekennzeichnet, dass** die Antikörper einzeln durch Immunisieren mit Antigenen erhalten werden, die von einem Salmonella-Stamm abgeleitet sind, der Lipopolysaccharid (LPS) aufweist, das O-spezifische Polysaccharide (OPS) enthält, die anders als O-spezifische Polysaccharide (OPS) von Lipopolysaccharid (LPS) des inaktivierten Salmonella sp.-Stamms sind,
und **dass** das Präparat den inaktivierten Salmonella sp.-Stamm und die Antikörper, die an den inaktivierten Salmonella sp.-Stamm gebunden sind, enthält, wobei die Antikörper in einer Menge von 25-100 mg pro Bakterienmasse von Salmonella in einer Konzentration von 2-5x10¹³ MPN (der wahrscheinlichsten Anzahl) vorliegen, wobei die Kombinationen von inaktiviertem Salmonella sp.-Stamm mit daran gebundenen Antikörpern ausgewählt wird unter inaktivierten Bakterien der O:4-Gruppe, die an Anti-O:9-Immunglobuline gebunden sind, die durch Immunisieren eines Wirts unter Anwendung von Salmonella aus der O:9-Gruppe hergestellt werden und/oder inaktivierten Bakterien der O:9-Gruppe, die an Anti-O:4-Immunglobuline gebunden sind, die durch Immunisieren eines Wirts unter Anwendung von Salmonella aus der O:4-Giüppc hergestellt werden, und/oder inaktivierten Bakterien der O:6,7-Gruppe, die an Anti-O:6,8-Immunglobuline gebunden sind, die durch Immunisieren eines Wirts unter Anwendung von Salmonella aus der O: 6,8-Gruppe hergestellt werden, und/oder inaktivierten Bakterien der O:6,8-Gruppe, die an Anti-O:6,7-Immunglobuline gebunden sind, die durch Immunisieren eines Wirts unter Anwendung von Salmonella aus der O:6,7-Gruppe hergestellt werden, wie beispielsweise inaktivierten Bakterien von S. Enteritidis O:9, die an Immunglobuline gebunden sind, die durch Immunisieren eines Wirts unter Anwendung von S. Paratyphi O:4 hergestellt werden, und/oder inaktivierten Bakterien von S. Thyphimurium O:4, die an Immunglobuline gebunden sind, die durch Immunisieren eines Wirts unter Anwendung von S. Katemba O:9 hergestellt werden, und/oder inaktivierten Bakterien von S. Hadar O:6,8, die an Immunglobuline gebunden sind, die durch Immunisieren eines Wirts unter Anwendung von S. Choleraesuis 0:6,7 hergestellt werden, und/oder inaktivierten Bakterien von S. Virchow O:6,7, die an Immunglobuline gebunden sind, die durch Immunisieren eines Wirts unter Anwendung von S. Newport O:6,8 hergestellt werden, und/oder inaktivierten Bakterien von S. Infantis O:6,7, die an Immunglobuline gebunden sind, die durch Immunisieren eines Wirts unter Anwendung von S. Newport O:6,8 hergestellt werden.

2. Präparat nach Anspruch 1, **dadurch gekennzeichnet, dass** die Antikörper nach der Immunisierung von Tieren mit einer Suspension von durch Sieden inaktivierten Bakterien erhalten werden.

3. Präparat nach Anspruch 1 oder 2, **dadurch gekennzeichnet, dass** es inaktivierte Bakterien der Salmonella-Gattung der Enterica-Spezies, Subspezies: Enterica salamae, arizonae und/oder diarizonae, houtenae, indica umfasst.

4. Präparat nach irgendeinem der Ansprüche 1-3, **dadurch gekennzeichnet, dass** die inaktivierten Bakterien der Salmonella-Gattung zu mindestens einer der folgenden Serogruppen gehören:
aus der Gruppe 4 (B), insbesondere Serovaren: Salmonella Typhimurium, Salmonella Typhimurium (z.B. 1,4, [5], 12: i:-), Salmonella agona, Salmonella bredeney, Salmonella heidelberg, Salmonella indiana, Salmonella Saint-Paul, Salmonella brandenburg, Salmonella agama, Salmonella derby, Salmonella stanley, Salmonella paratyphi B, Salmonella schleissheim, Salmonella chester;
aus der Gruppe 6,7 und 8,20 (C1 - C3), insbesondere Serovare: Salmonella paratyphi C, Salmonella infantis, Salmonella hadar, Salmonella mbandaka, Salmonella livingstone, Salmonella virchow, Salmonella ohio, Salmonella montevideo, Salmonella tennessee, Salmonella rissen, Salmonella bareilly, Salmonella oranienburg, Salmonella thompson, Salmonella newport, Salmonella kottbus, Salmonella kentucky, Salmonella albany, Salmonella bovismorbificans, Salmonella djugu, Salmonella breanderup, Salmonella jeruzalem, Salmonella chester, Salmonella goldcoast, Salmonella braenderup;
aus der Gruppe 9 und 9,46 (D1 - D2), insbesondere Serovare: Salmonella enteritidis, Salmonella panama, Salmonella dublin, Salmonella gallinarum, Salmonella miami, Salmonella sendai, Salmonella Wernigerode, Salmonella typhi;
aus der Gruppe 3,10 [15, 34] und 1,3,19 (E1 - E4), insbesondere Serovare: Salmonella give, Salmonella anatum, Salmonella london, Salmonella meleagridis, Salmonella Cambridge, Salmonella minneapolis, Salmonella simsbury, Salmonella senftenberg, Salmonella westerstede;
aus der Gruppe 11 (F), insbesondere Salmonella aberdeen;
aus der Gruppe 13 (G1 - G2), insbesondere Serovare: Salmonella cubana, Salmonella poona;
aus der Gruppe 6,14 (H), insbesondere Serovare: Salmonella heves, Salmonella onderstepoort;
aus der Gruppe 16 (I), insbesondere Serovare: Salmonella brazil, Salmonella hvittingfoss;
aus der Gruppe 17 (J), insbesondere Serovar Salmonella berlin;
aus der Gruppe 18 (K), insbesondere Serovar Salmonella illa (0:18);
und ferner den Serogruppen, die 21, 28, 30, 35, 38, 39, 40, 41, 42, 43, 44, 45, 47, 48 und 50 nummeriert sind.

5. Präparat nach irgendeinem der Ansprüche 1-4, **dadurch gekennzeichnet, dass** die Antikörper aus Serum eines Kaninchen isoliert sind, das mit Salmonella-Antigenen immunisiert worden ist.

6. Präparat nach irgendeinem der Ansprüche 1-4, **dadurch gekennzeichnet, dass** die Antikörper aus Vogeleigelb, insbesondere von einem Hühnerei, von einem Vogel, der mit Salmonella-Antigenen immunisiert worden ist, isoliert werden.

7. Präparat nach irgendeinem der Ansprüche 1-4, **dadurch gekennzeichnet, dass** es Antikörper enthält, die von Serum eines Kaninchen isoliert worden sind, das mit Salmonella-Antigenen immunisiert worden ist, und Antikörper, die von Eigelb eines Vogels, insbesondere eines Huhns, der/das mit Salmonella-Antigenen immunisiert worden ist, isoliert sind.

8. Präparat nach irgendeinem der Ansprüche 1 bis 7, **dadurch gekennzeichnet, dass** es Antikörper in einer Menge von 50 mg/2-5x10¹³ von Bakterien enthält.

9. Präparat nach irgendeinem der Ansprüche 1 bis 8 zur Verwendung als pharmazeutische Zusammensetzung, insbesondere als Impfstoff.

10. Präparat nach irgendeinem der Ansprüche 1 bis 8 zur Verwendung bei der Immunisierung und/oder der Behandlung von Salmonellose bei Tieren, insbesondere Vögeln.

11. Verfahren zum Herstellen des mehrwertigen, kombinierten immunisierenden und/oder therapeutischen Präparats nach irgendeinem der Ansprüche 1 bis 8, **dadurch gekennzeichnet, dass** es die Schritte umfasst, wobei:
a) die Antikörper einzeln durch Immunisieren eines Wirts mit ausgewähltem Salmonella-Antigen erhalten werden;
b) eine sterile Bakteriensuspension getrennt aus einem ausgewählten Bakterienstamm von Salmonella hergestellt wird;
c) zu jeder sterilen Bakteriensuspension, die in Schritt b) hergestellt worden ist, Antikörper zugegeben werden, die in Schritt a) erhalten worden sind, wobei die Antikörper aus Schritt a) durch Immunisieren mit Antigenen erhalten worden sind, die von dem Salmonella-Stamm abgeleitet sind, der Lipopolysaccharid (LPS) aufweist, das O-spezifische Polysaccharide (OPS) enthält, die anders sind als O-spezifische Polysaccharide (OPS) von Lipopolysaccharid (LPS) der Bakterien der sterilen Bakteriensuspension aus Schritt b), um spontanes Binden von Antikörpern an nichtspezifische O-Polysaccharidfraktionen bereitzustellen und wobei die Antikörper in einer Menge von 25-100 mg pro Bakterienmasse von Salmonella in einer Konzentration von 2-5x10¹³ MPN, bevorzugt 50 mg/2-5x10¹³ Bakterien zugegeben werden;
d) inaktivierte Bakterien mit Antikörpern, die daran adsorbiert sind, zusammengemischt werden;
e) die erhaltene Suspension zentrifugiert wird und das resultierende Kügelchen erneut in einer physiologischen Kochsalzlösung wahlweise mit Zusatz eines Konservierungsmittels, bevorzugt Formaldehyd oder Phenol, oder einer Mischung davon, in einer Konzentration von nicht mehr als 0,05 % suspendiert wird;
f) das erhaltene Präparat mit an Salmonella-Bakterien absorbierten Antikörpern vom Reaktionsmedium durch Zentrifugieren isoliert wird, wobei in Schritt c) inaktivierte Bakterien der O:4-Gruppe mit Anti-O:9-Immunglobulinen gemischt und daran gebunden werden, die durch Immunisieren eines Wirts unter Anwendung von Salmonella aus der O:9-Gruppe hergestellt werden, und/oder inaktivierte Bakterien der O:9-Gruppe mit Anti-O:4-Immunglobulinen gemischt und daran gebunden werden, die durch Immunisieren eines Wirts unter Anwendung von Salmonella aus der O:4-Gruppe hergestellt werden, und/oder inaktivierte Bakterien der O:6,7-Gruppe mit Anti-O:6,8-Immunglobulinen gemischt und daran gebunden werden, die durch Immunisieren eines Wirts unter Anwendung von Salmonella aus der O:6,8-Gruppe hergestellt werden, und/oder inaktivierte Bakterien der O:6,8-Gruppe mit Anti-O:6,7-Immunglobulinen gemischt und daran gebunden werden, die durch Immunisieren eines Wirts unter Anwendung von Salmonella aus der O:6,7-Gruppe hergestellt werden, wie beispielsweise inaktivierte Bakterien von S. Enteritidis O:9 mit Immunglobulinen gemischt und daran gebunden werden, die durch Immunisieren eines Wirts unter Anwendung von S. Paratyphi B O:4 hergestellt werden, und/oder inaktivierte Bakterien von S. Thyphimurium O:4 mit Immunglobulinen gemischt und daran gebunden werden, die durch Immunisieren eines Wirts unter Anwendung von S. Katemba O:9 hergestellt werden, und/oder inaktivierte Bakterien von S. Hadar O:6,8 mit Immunglobulinen gemischt und daran gebunden werden, die durch Immunisieren eines Wirts unter Anwendung von S. Choleraesuis 0:6,7 hergestellt werden, und/oder inaktivierte Bakterien von S. Virchow O:6,7, die mit Immunglobulinen gemischt und daran gebunden werden, die durch Immunisieren eines Wirts unter Anwendung von S. Newport O:6,8 hergestellt werden, und/oder inaktivierte Bakterien von S. Infantis 0:6,7 mit Immunglobulinen gemischt und daran gebunden werden, die durch Immunisieren eines Wirts unter Anwendung von S. Newport O:6,8 hergestellt werden.

12. Verfahren nach Anspruch 11, **dadurch gekennzeichnet, dass** die sterile Bakterienmasse mit Antikörpern gemischt wird, die aus Sera von Kaninchen, die mit von Salmonella abgeleiteten Antigenen immunisiert worden sind, erhalten werden.

13. Verfahren nach Anspruch 11, **dadurch gekennzeichnet, dass** die sterile Bakterienmasse mit Antikörpern gemischt wird, die aus Eigelb von Vögeln abgeleitet sind, die mit von Salmonella abgeleiteten Antigenen immunisiert worden sind.

14. Verfahren nach Anspruch 11, **dadurch gekennzeichnet, dass** die sterile Bakterienmasse mit Antikörpern gemischt wird, die aus Sera von Kaninchen und Eigelb von Vögeln, die mit von Salmonella abgeleiteten Antigenen immunisiert worden sind, erhalten werden.

15. Verfahren nach irgendeinem der Ansprüche 11 bis 14, **dadurch gekennzeichnet, dass** die Konzentration von Bakterien in dem Präparat nicht weniger als 10¹² Bakterien/ml und nicht mehr 10¹⁴ Bakterien/ml beträgt.

16. Verwendung des Präparats nach Anspruch 1 bis 8 für die Herstellung eines Impfstoffs gegen Salmonellose bei Tieren, insbesondere Vögeln.

17. Anti-Salmonellose-Impfstoff, **dadurch gekennzeichnet, dass** er ein Präparat nach irgendeinem der Ansprüche 1 bis 8 und einen pharmazeutisch akzeptablen Träger umfasst.

18. Impfstoff nach Anspruch 17 zur Verwendung bei der Immunisierung und/oder Behandlung von Salmonellose bei Tieren, insbesondere Vögeln, der zur Verabreichung in Trinkwasser bestimmt ist.

19. Impfstoff zur Verwendung nach Anspruch 18, der zur Verabreichung an junge Individuen vom ersten Tag ihres Lebens an bestimmt ist.

20. Impfstoff zur Verwendung nach Anspruch 18 oder 19, welche Verabreichung 3- oder viermal, bevorzugt alle 7 bis 10 Tage, wiederholt wird.

## Revendications

1. Une préparation polyvalente, combinée, immunisante et/ou thérapeutique contre la salmonellose comprenant une souche de *Salmonella sp.* inactivée avec des anticorps liés à celle-ci, **caractérisée en ce que** lesdits anticorps sont obtenus séparément par immunisation avec des antigènes dérivés de la souche *Salmonella* ayant un lipopolysaccharide (LPS) contenant des polysaccharides à spécificité O (OPS) qui sont différents de polysaccharides à spécificité O (OPS) de lipopolysaccharide (LPS) de ladite souche de *Salmonella sp.* inactivée,
et **en ce que** la préparation contient ladite souche de *Salmonella sp.* inactivée et lesdits anticorps, liés à ladite souche de *Salmonella sp.* inactivée, les anticorps étant présents en une quantité de 25 à 100 mg par masse bactérienne de *Salmonella* en concentration de 2 à 5x10¹³ MPN (nombre le plus probable), la combinaison de la souche *Salmonella sp.* inactivée. avec des anticorps liés à elle étant choisie parmi les bactéries inactivées du groupe O:4 liées aux immunoglobulines anti-O:9 produites en immunisant un hôte par l'utilisation de *Salmonella* du groupe O:9, et/ou des bactéries inactivées du groupe O:9 liées aux immunoglobulines anti-O:4 produites en immunisant un hôte par l'utilisation de *Salmonella* du groupe O:4, et/ou des bactéries inactivées du groupe 0:6,7 liées aux immunoglobulines anti-O:6,8 produites en immunisant un hôte par l'utilisation de *Salmonella* du groupe O:6.8 et/ou des bactéries inactivées du groupe O:6,8 liées à des immunoglobulines anti-O:6,7 produites en immunisant un hôte par l'utilisation de *Salmonella* du groupe O:6,7, telles que des bactéries inactivées de S. Enteritidis O:9 liées à des immunoglobulines produites en immunisant un hôte par l'utilisation de *S*. *Paratyphi B* O:4, et/ou des bactéries inactivées de *S*. Typhimurium O:4 liées à des immunoglobulines produites en immunisant un hôte par l'utilisation de *S*. Kapemba O:9, et/ou bactéries inactivées de *S.* Hadar O:6,8 liées à des immunoglobulines produites en immunisant un hôte par l'utilisation de *S*. Choleraesuis O:6,7, et/ou des bactéries inactivées de *S*. Virchow 0:6,7 liées à des immunoglobulines produites en immunisant un hôte par l'utilisation de *S.* Newport O:6,8, et/ou des bactéries inactivées de *S.* Infantis 0:6,7 liées à des immunoglobulines produites en immunisant un hôte par l'utilisation de *S*. Newport O:6,8.

2. La préparation selon la revendication 1, **caractérisée en ce que** lesdits anticorps sont obtenus après immunisation d'animaux avec une suspension de bactéries inactivées par ébullition.

3. La préparation selon la revendication 1 ou la revendication 2, **caractérisée en ce qu'**elle comprend des bactéries inactivées du genre *Salmonella* espèce *enterica,* sous-espèces : *enterica, salamae, arizonae* et/ou *diarizonae, houtenae, Indica.*

4. La préparation selon l'une quelconque des revendications 1 à 3, **caractérisée en ce que** les bactéries inactivées du genre *Salmonella* appartiennent à au moins un des sérogroupes suivants :
Du groupe 4 (B), en particulier les sérotypes : *Salmonella* Typhimurium, *Salmonella* Typhimurium (par exemple, 1,4,[5], 12 : i : -), *Salmonella* Agona, *Salmonella* Bredeney, *Salmonella* Heidelberg, *Salmonella* Indiana, *Salmonella* Saintpaul, *Salmonella* Brandenburg, *Salmonella* Agama, *Salmonella* Derby, *Salmonella* Stanley, *Salmonella* Paratyphi B, *Salmonella* Schleissheim, *Salmonella* Chester ;
Du groupe 6,7 et 8,20 (C1 - C3), en particulier les sérotypes : *Salmonella* Paratyphi C, *Salmonella* Infantis, *Salmonella* Hadar, *Salmonella* Mbandaka, *Salmonella* Livingstone, *Salmonella* Virchow, *Salmonella* Ohio, *Salmonella* Montevideo, *Salmonella* Tennessee, *Salmonella* Rissen, *Salmonella* Bareilly, *Salmonella* Oranienburg, *Salmonella* Thompson, *Salmonella* Newport, *Salmonella* Kottbus, *Salmonella* Kentucky, *Salmonella* Albany, *Salmonella* Bovismorbificans, *Salmonella* Djugu, *Salmonella* Breanderup, *Salmonella* Jeruzalem, *Salmonella* Chester, *Salmonella* Goldcoast et *Salmonella* Braenderup ;
Du groupe 9 et 9,46 (D1 - D2), en particulier les sérotypes : *Salmonella* Enteritidis, *Salmonella* Panama, *Salmonella* Dublin, *Salmonella* Gallinarum, *Salmonella* Miami, *Salmonella* Sendai, *Salmonella* Wernigerode, *Salmonella* Typhi ;
Du groupe 3,10[15, 34] et 1,3,19 (E1 - E4), en particulier les sérovars : *Salmonella* Give, *Salmonella* Anatum, *Salmonella* London, *Salmonella* Meleagridis, *Salmonella* Cambridge, *Salmonella* Minneapolis, *Salmonella* Simsbury, *Salmonella* Senftenberg, *Salmonella* Westerstede ;
Du groupe 11 (F), en particulier *Salmonella* Aberdeen ;
Du groupe 13 (G1 - G2), en particulier les sérotypes : *Salmonella* Cubana, *Salmonella* Poona;
Du groupe 6,14 (H), en particulier les sérotypes : *Salmonella* Heves, *Salmonella* Onderstepoort ;
Du groupe 16 (1), en particulier les sérotypes : *Salmonella* Brazil, *Salmonella* Hvittingfoss ;
Du groupe 17 (J), en particulier le sérotype *Salmonella* Berlin ;
Du groupe 18 (K), en particulier le sérotype *Salmonella* IIIa sérotype (O:18) ;
et d'autres sérogroupes numérotés 21, 28, 30, 35, 38, 39, 40, 41, 42, 43, 44, 45, 47, 48 et 50.

5. La préparation selon l'une quelconque des revendications 1 à 4, **caractérisée en ce que** lesdits anticorps sont isolés à partir du sérum d'un lapin immunisé avec des antigènes de *Salmonella.*

6. La préparation selon l'une quelconque des revendications 1 à 4, **caractérisée en ce que** lesdits anticorps sont isolés à partir d'un jaune d'oeuf d'oiseau, en particulier d'un oeuf de poule, provenant d'un oiseau immunisé avec des antigènes de *Salmonella.*

7. La préparation selon l'une quelconque des revendications 1 à 4, **caractérisée en ce qu'**elle contient des anticorps isolés à partir du sérum d'un lapin immunisé avec des antigènes de *Salmonella* et des anticorps isolés à partir du jaune d'oeuf d'un oiseau, en particulier la poule, immunisé avec des antigènes de *Salmonella.*

8. La préparation selon l'une quelconque des revendications 1 à 7, **caractérisée en ce qu'**elle contient des anticorps en une quantité de 50 mg/2 à 5x10¹³ de bactéries.

9. La préparation selon l'une quelconque des revendications 1 à 8 pour utilisation en tant que composition pharmaceutique, en particulier en tant que vaccin.

10. La préparation selon l'une quelconque des revendications 1 à 8 pour utilisation dans l'immunisation et/ou le traitement des salmonelloses chez les animaux, en particulier les oiseaux.

11. Un procédé de production de la préparation polyvalente, combinée, immunisante et/ou thérapeutique selon l'une quelconque des revendications 1 à 8, **caractérisé en ce qu'**il comprend les étapes dans lesquelles :
a) des anticorps sont obtenus séparément en immunisant un hôte avec un antigène *Salmonella* sélectionné ;
b) une suspension bactérienne stérile est préparée séparément d'une souche bactérienne sélectionnée de *Salmonella ;*
c) à chaque suspension bactérienne stérile préparée à l'étape b), on ajoute des anticorps obtenus à l'étape a), les anticorps de l'étape a) ayant été obtenus par immunisation avec des antigènes dérivés de la souche *Salmonella* ayant un lipopolysaccharide (LPS) contenant des polysaccharides (OPS) à spécificité O qui sont différents des polysaccharides à spécificité O (OPS) du lipopolysaccharide (LPS) des bactéries de ladite suspension bactérienne stérile résultant de l'étape b), pour fournir une liaison spontanée d'anticorps à des fractions de polysaccharide non à spécificité O, et les anticorps étant ajoutés en une quantité de 25 à 100 mg par masse bactérienne de *Salmonella* à une concentration de 2 à 5x10¹³ MPN, de préférence de 50 mg/2 à 5x10¹³ de bactéries ;
d) les bactéries inactivées avec les anticorps adsorbés par elles sont mélangés ensemble ;
e) la suspension obtenue est centrifugée et le granule obtenu est remis en suspension dans une solution physiologique saline, éventuellement avec addition d'un conservateur, de préférence du formaldéhyde ou du phénol, ou d'un mélange de ceux-ci, à une concentration non supérieure à 0,05 % ;
f) la préparation obtenue avec des anticorps absorbés sur les bactéries *Salmonella* est isolée du milieu réactionnel par centrifugation ; dans l'étape c), des bactéries inactivées du groupe O:4 sont mélangées et liées à des anti-immunoglobulines O:9 produites en immunisant un hôte par l'utilisation de *Salmonella* du groupe O:9,et/ou des bactéries inactivées du groupe O:9 sont mélangées et liées à des anti-immunoglobulines O:4 produites en immunisant un hôte par l'utilisation de *Salmonella* du groupe O:4, et/ou des bactéries inactivées du groupe O:6.7 sont mélangées et liées à des anti- immunoglobulines O:6.8 produites en immunisant un hôte par l'utilisation de *Salmonella* du groupe O:6.8, et/ou des bactéries inactivées du groupe O:6,8 sont mélangées et liées à des immunoglobulines anti-O:6,7 produites en immunisant un hôte par l'utilisation de *Salmonella* du groupe O:6,7, telles que des bactéries inactivées de *S. Enteritidis* O:9 mélangées et liées à des immunoglobulines produites en immunisant un hôte par l'utilisation de *S. Paratyphi B* O:4, et/ou des bactéries inactivées de *S*. *Typhimurium* O:4 sont mélangées et liées à des immunoglobulines produites en immunisant un hôte par l'utilisation de *S. Kapemba* O:9, et/ou des bactéries inactivées de *S. Hadar* O:6,8 sont mélangées et liées à des immunoglobulines produites en immunisant un hôte par l'utilisation de *S. Choleraesuis* O:6,7, et/ou des bactéries inactivées de *S. Virchow* O:6,7 sont mélangées et liées à des immunoglobulines produites en immunisant un hôte par l'utilisation de *S. Newport* O:6,8, et/ou des bactéries inactivées de *S*. *Infantis* O:6,7 sont mélangées et liées à des immunoglobulines produites en immunisant un hôte par l'utilisation de *S. Newport* O:6,8.

12. Le procédé selon la revendication 11 **caractérisé en ce que** la masse bactérienne stérile est mélangée à des anticorps obtenus à partir de sérums de lapins immunisés avec des antigènes dérivés de *Salmonella.*

13. Le procédé selon la revendication 11, **caractérisé en ce que** la masse bactérienne stérile est mélangée à des anticorps dérivés du jaune d'oeuf d'oiseaux immunisés avec des antigènes dérivés de *Salmonella.*

14. Le procédé selon la revendication 11, **caractérisé en ce que** la masse bactérienne stérile est mélangée à des anticorps obtenus à partir de sérums de lapins et de jaunes d'oeufs d'oiseaux immunisés avec des antigènes dérivés de *Salmonella.*

15. Le procédé selon l'une quelconque des revendications 11 à 14, **caractérisé en ce que** la concentration de bactéries dans la préparation n'est pas inférieure à 10¹² bactéries/ml et pas supérieure à 10¹⁴ bactéries/ml.

16. Utilisation de la préparation selon l'une des revendications 1 à 8 pour la production d'un vaccin contre les salmonelloses chez les animaux, en particulier les oiseaux.

17. Un vaccin contre la salmonellose, **caractérisé en ce qu'**il comprend une préparation selon l'une quelconque des revendications 1 à 8 et un support pharmaceutiquement acceptable.

18. Le vaccin selon la revendication 17 à utiliser pour l'immunisation et/ou le traitement des salmonelloses chez les animaux, en particulier les oiseaux, qui est destiné à être administré dans l'eau potable.

19. Le vaccin à utiliser selon la revendication 18, qui est destiné à être administré aux jeunes individus dès le premier jour de leur vie.

20. Le vaccin à utiliser selon la revendication 18 ou la revendication 19, dont l'administration est répétée 3 ou 4 fois, de préférence tous les 7 à 10 jours.
